(19) Europäisches Patentamt
European Patent Office
Office européen des brevets

(11) Publication number: **0 556 024 B1**

(12) # EUROPEAN PATENT SPECIFICATION

(45) Date of publication of patent specification: **02.08.95**

(51) Int. Cl.6: **C07D 307/54**, C07D 307/85, A61K 31/34

(21) Application number: **93300938.3**

(22) Date of filing: **09.02.93**

(54) **Novel amidinonaphthyl furancarboxylate derivatives and acid addition salts thereof.**

(30) Priority: **10.02.92 JP 23848/92**

(43) Date of publication of application:
**18.08.93 Bulletin 93/33**

(45) Publication of the grant of the patent:
**02.08.95 Bulletin 95/31**

(84) Designated Contracting States:
**AT BE CH DE DK FR GB IT LI LU NL SE**

(56) References cited:
**US-A- 4 563 527**
**US-A- 4 634 783**

(73) Proprietor: **TORII & CO., LTD.**
**4-1, Nihonbashi-Honcho-3-chome**
**Chuo-ku**
**Tokyo (JP)**

(72) Inventor: **Nakayama, Toyoo**
**61-2-306, Shibayama-6-chome**
**Funabashi-shi (JP)**
Inventor: **Taira, Seizo**
**19-10, Saiwaicho-2-chome**
**Mihama-ku,**
**Chiba-shi (JP)**
Inventor: **Kawamura, Hiroyuki**
**18-19-508, Kakemama-2-chome**
**Ichikawa-shi (JP)**
Inventor: **Shibuya, Masaoki**
**18-1, Kotehashidai-3-chome**
**Hanamigawa-ku,**
**Chiba-shi (JP)**
Inventor: **Iwaki, Masahiro**
**10-14, Kitakokubun-3-chome**
**Ichikawa-shi (JP)**

(74) Representative: **Silveston, Judith et al**
**ABEL & IMRAY**
**Northumberland House**
**303-306 High Holborn**
**London, WC1V 7LH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to novel amidinonaphthyl furancarboxylate derivatives and acid addition salts thereof, and pharmaceutical compositions containing them.

Various autoimmune diseases are known, including, for example, systemic lupus erythematosus, articular rheumatism, scleroderma, etc. Pharmaceuticals generally known at present for treating these diseases are steroidal agents. Steroidal agents are very difficult to use properly. For example, erroneous use of steroidal agents causes various side effects due to the lowering of immunological functions or the atrophy of the adrenal gland, and sometimes ultimately causes death.

It is generally said that nephritis and autoimmune diseases are closely related with proteases and complement.

Amidinonaphthyl furancarboxylate derivatives are known to have activities of inhibiting enzymes such as trypsin, plasmin, kallikrein, thrombin, etc., and of inhibiting complement. It has been suggested that these enzyme inhibitors are useful as agents for the treatment of pancreatitis, agents for the treatment of hemorrhagic diseases, agents for the treatment of thrombosis or agents for the treatment of nephritis (Japanese Patent Kokai (Laid-open) Nos. 59-139357 and 61-22075).

However, though these compounds all show strong antienzyme activities, they are not yet satisfactory in their effectiveness and safety.

The object of this invention is to provide compounds which strongly inhibit the activity of trypsin, plasmin, thrombin, kallikrein or complement, exert no strong side effect unlike steroidal agents, and exhibit a stronger efficacy in treating nephritis and autoimmune diseases than the known compounds mentioned above.

The compounds disclosed in Japanese Patent Kokai (Laid-open) Nos. 59-139357 and 61-22075 all contain neither carboxylic acid group nor its derivative at the terminal opposite to the amidinonaphthol group.

The present inventors converted the terminal portion of such compounds opposite to the amidinonaphthol group into a carboxylic acid or its derivative. Thus, this invention relates to amidinonaphthyl furancarboxylate derivatives of the formula I

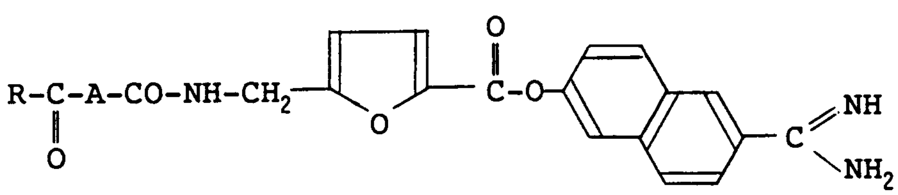

I

wherein A is a single bond or A denotes

a) a phenyl group, a cyclopentyl group or a cyclohexyl group;

b) an alkenylphenyl group, an alkylphenyl group, a phenylalkenyl group or a phenylalkyl group wherein alkyl is $C_1$ to $C_7$ alkyl and alkenyl is $C_2$ to $C_7$ alkenyl;

c) a $C_1$ to $C_{18}$ alkyl or $C_2$ to $C_7$ alkenyl group, which may be substituted by one or two substituents selected from $C_1$ to $C_5$ alkyl groups and guanidino groups, wherein an alkyl substituent together with the carbon atom to which it is attached, may form a cycloalkyl ring having from 3 to 6 carbon atoms, or a $C_1$ to $C_5$ alkyl may itself be substituted by a cycloalkyl ring; or

d) $-(CH_2)_n-NH-CO-(CH_2)_{n'}-$ wherein n and n', which may be the same or different, each represent an integer from 1 to 4; and

R denotes a hydroxyl group; a $C_1$ to $C_7$ alkoxy group which may be mono- or di-substituted by $C_1$ to $C_5$ alkyl; a phenyl-$C_1$-$C_4$alkoxy group; $-OAl(OH)_2$; an amino group;

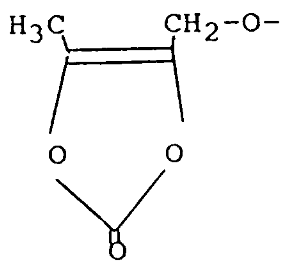 or 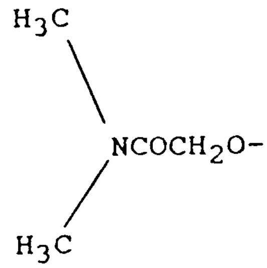

or a pharmaceutically acceptable acid addition salt thereof.

In the compounds of the present invention R is preferably a hydroxyl group, an amino group or a $C_1$ to $C_7$ alkoxy group which may be mono- or di-substituted by $C_1$ to $C_5$ alkyl.

Examples of compounds of formula I are those in which:

1) A is a $C_2$ to $C_7$alkenylphenyl group and R is a hydroxyl group or a $C_1$ to $C_7$ alkoxy group which may be mono- or di-substituted by $C_1$ to $C_5$ alkyl;

2) A is a phenyl-$C_1$-$C_7$alkyl group and R is a hydroxyl group or a $C_1$ to $C_7$ alkoxy group which may be mono- or di-substituted by $C_1$ to $C_5$ alkyl;

3) A is a $C_1$ to $C_{18}$ alkyl or $C_2$ to $C_7$ alkenyl group, which may be substituted by one or two substituents selected from $C_1$ to $C_5$ alkyl groups and guanidino groups, wherein an alkyl substituent together with the carbon atom to which it is attached, may form a cycloalkyl ring having from 3 to 6 carbon atoms, or an $C_1$ to $C_5$ alkyl may itself be substituted by a cycloalkyl ring, and R is a hydroxyl group, $C_1$ to $C_7$ alkoxy group which may be mono-or di-substituted by $C_1$ to $C_5$ alkyl, $-OAl(OH)_2$, amino group,

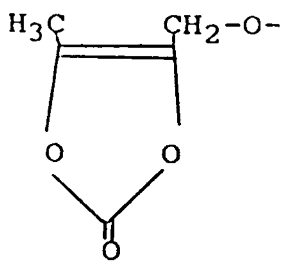 or 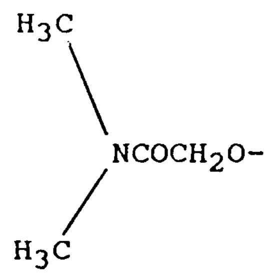 ;

4) A is an $C_1$-$C_7$alkylphenyl group and R is a hydroxyl group or a $C_1$ to $C_7$ alkoxy group which may be mono- or di-substituted by $C_1$ to $C_5$ alkyl;

5) A is a phenyl group and R is a hydroxyl group;

6) A is a phenyl-$C_2$-$C_7$alkenyl group and R is a hydroxyl group;

7) A is a cyclopentyl group or a cyclohexyl group and R is a hydroxyl group;

8) A is $-(CH_2)_n$-NH-CO-$(CH_2)_{n'}$- wherein n and n', which may be the same or different, each represent an integer from 1 to 4, and R is a hydroxyl group or a $C_1$ to $C_7$ alkoxy group which may be mono- or di-substituted by $C_1$ to $C_5$ alkyl or a phenyl-$C_1$-$C_4$alkoxy group,

9) A is a single bond and R is a hydroxyl group or a $C_1$ to $C_7$, advantageously $C_1$ to $C_4$, alkoxy group which may be mono- or di-substituted by $C_1$ to $C_5$ alkyl;

10) A is a $C_1$ to $C_{18}$ alkyl group and R is a hydroxyl group, amino group or a $C_1$ to $C_7$ alkoxy group which may be mono- or di-substituted by $C_1$ to $C_5$ alkyl.

The compounds of this invention strongly inhibit the activity of trypsin, plasmin, thrombin, kallikrein or complement. These compounds having protease inhibitory activities can be used, for example, as a stabilizer for preventing the decomposition of collected blood due to these enzymatic activities, in the determination of fibrinopeptide etc. relating to the blood coagulation system. Accordingly, the present invention provides for the use <u>in vitro</u> of a compound according to the invention as an enzyme inhibitor, especially where the enzyme is trypsin, plasmin, kallikrein, thrombin or complement. The invention also provides a method for preventing the decomposition of collected blood, which comprisies admixing a compound according to the invention with the collected blood.

Anti-trypsin, Anti-plasmin, Anti-kallikrein and Anti-thrombin Activities.

Table 1 shows the inhibitory activities of representative compounds of this invention against trysin, plasmin, complement, kallikrein and thrombin. The values in Table 1 represent molar concentrations ($IC_{50}$) of test compounds which inhibit 50% of the activity of each enzyme to hydrolyze TAME (tosylarginine methyl ester). The anti-trypsin, anti-plasmin, anti-kallikrein and anti-thrombin activities were determined according to the method of Muramatsu et al. (J. Biochem., 58, 214 (1965)).

Anti-complement Activity

The anti-C1 esterase ($C1\bar{r}$, $C1\bar{s}$) activity was determined according to the method of Okamura et al. (Biochem. Biophys. Acta, 295, 252-257 (1973)). The values for $C1\bar{r}$ in the Table I represent molar concentrations ($IC_{50}$) of test compounds which inhibit 50% of the activity of $C1\bar{r}$ to hydrolyze AAME (acetylarginine methyl ester). The values for $C1\bar{s}$ represent molar concentrations ($IC_{50}$) of test compounds which inhibit 50% of the activity of $C1\bar{s}$ to hydrolyze ATEE (acetyltyrosine ethyl ester).

Table 1 shows the anti-trypsin, anti-plasmin, anti-kallikrein, anti-thrombin and anti-complement activities of representative compounds of this invention.

Table 1

| Example No. | Try | Pla | $Cl\bar{r}$ | $Cl\bar{s}$ | Kal | Thr |
|---|---|---|---|---|---|---|
| 1 | $3.5x10^{-7}$ | $1.5x10^{-7}$ | $3.4x10^{-7}$ | $2.7x10^{-7}$ | $4.7x10^{-7}$ | $3.5x10^{-7}$ |
| 2 | $8.4x10^{-6}$ | $4.8x10^{-6}$ | $29(10^{-5})$ | $17(10^{-5})$ | $46(10^{-5})$ | $47(10^{-5})$ |
| 3 | $48(10^{-5})$* | $8.6x10^{-6}$ | $34(10^{-5})$ | $3.4x10^{-6}$ | $33(10^{-5})$ | $26(10^{-5})$ |
| 4 | $3.4x10^{-7}$ | $7.2x10^{-8}$ | $6.4x10^{-8}$ | $1.6x10^{-7}$ | $2.1x10^{-7}$ | $7.0x10^{-8}$ |
| 5 | $3.4x10^{-7}$ | $1.0x10^{-7}$ | $7.3x10^{-8}$ | $3.6x10^{-7}$ | $3.0x10^{-7}$ | $1.8x10^{-7}$ |
| 6 | $3.9x10^{-7}$ | $2.6x10^{-7}$ | $2.3x10^{-8}$ | $2.7x10^{-7}$ | $8.3x10^{-7}$ | $1.0x10^{-5}$ |
| 7 | $1.8x10^{-6}$ | $3.0x10^{-7}$ | $7.4x10^{-7}$ | $4.6x10^{-7}$ | $1.5x10^{-6}$ | $4.0x10^{-7}$ |
| 8 | $2.1x10^{-6}$ | $1.1x10^{-6}$ | $8.3x10^{-7}$ | $2.0x10^{-6}$ | $2.7x10^{-6}$ | $1.4x10^{-6}$ |
| 9 | $4.8x10^{-6}$ | $5.0x10^{-7}$ | $2.8x10^{-6}$ | $2.3x10^{-6}$ | $6.0x10^{-6}$ | $6.5x10^{-7}$ |
| 10 | $6.0x10^{-7}$ | $1.5x10^{-7}$ | $1.6x10^{-7}$ | $2.6x10^{-8}$ | $2.5x10^{-7}$ | $1.6x10^{-6}$ |
| 11 | $6.6x10^{-7}$ | $4.0x10^{-7}$ | $1.2x10^{-6}$ | $2.8x10^{-7}$ | $8.7x10^{-7}$ | $1.2x10^{-6}$ |
| 12 | $4.8x10^{-7}$ | $2.7x10^{-7}$ | $2.8x10^{-6}$ | $2.1x10^{-7}$ | $7.7x10^{-7}$ | $6.0x10^{-7}$ |
| 15 | $7.1x10^{-7}$ | $3.1x10^{-7}$ | $8.4x10^{-7}$ | $2.3x10^{-7}$ | $3.2x10^{-7}$ | $4.0x10^{-7}$ |
| 16 | $3.4x10^{-7}$ | $2.4x10^{-7}$ | $6.9x10^{-6}$ | $3.5x10^{-7}$ | $3.8x10^{-6}$ | $1.3x10^{-6}$ |
| 17 | $4.7x10^{-7}$ | $4.3x10^{-8}$ | $1.7x10^{-6}$ | $5.3x10^{-8}$ | $1.1x10^{-6}$ | $1.9x10^{-6}$ |
| 18 | $4.0x10^{-7}$ | $1.9x10^{-7}$ | $3.4x10^{-7}$ | $1.9x10^{-7}$ | $2.1x10^{-7}$ | $3.5x10^{-7}$ |

| Example No. | Try | Pla | $Cl\bar{r}$ | $Cl\bar{s}$ | Kal | Thr |
|---|---|---|---|---|---|---|
| 19 | $2.5x10^{-7}$ | $1.7x10^{-7}$ | $1.8x10^{-6}$ | $7.2x10^{-7}$ | $2.5x10^{-7}$ | $3.6x10^{-7}$ |
| 20 | $2.0x10^{-6}$ | $1.8x10^{-7}$ | $3.8x10^{-7}$ | $3.9x10^{-7}$ | $1.3x10^{-7}$ | $4.6x10^{-7}$ |
| 21 | $3.1x10^{-7}$ | $3.3x10^{-7}$ | $5.4x10^{-7}$ | $1.8x10^{-7}$ | $7.0x10^{-7}$ | $9.6x10^{-7}$ |
| 22 | $4.3x10^{-7}$ | $4.3x10^{-7}$ | $49(10^{-5})$ | $2.5x10^{-7}$ | $4.7x10^{-7}$ | $3.5x10^{-6}$ |
| 27 | $3.1x10^{-7}$ | $3.3x10^{-7}$ | $5.4x10^{-7}$ | $1.8x10^{-7}$ | $7.0x10^{-7}$ | $9.6x10^{-7}$ |
| 29 | $1.2x10^{-6}$ | $2.3x10^{-7}$ | $4.4x10^{-7}$ | $3.6x10^{-7}$ | $2.4x10^{-7}$ | $1.6x10^{-6}$ |
| 31 | $6.0x10^{-7}$ | $1.6x10^{-7}$ | $2.9x10^{-7}$ | $4.5x10^{-7}$ | $1.7x10^{-7}$ | $1.5x10^{-7}$ |
| 33 | $6.6x10^{-6}$ | $8.4x10^{-8}$ | $1.4x10^{-7}$ | $2.2x10^{-7}$ | $5.8x10^{-7}$ | $2.3x10^{-8}$ |
| 34 | $2.7x10^{-7}$ | $1.3x10^{-7}$ | $3.3x10^{-7}$ | $1.7x10^{-7}$ | $1.3x10^{-7}$ | $8.9x10^{-8}$ |

Note: * Indicated in terms of percentage inhibition at a concentration of 1 x $10^{-5}$ M.

The present invention provides pharmaceutical preparations comprising the compounds of the invention optionally in combination with at least one pharmaceutically acceptable carrier.

The compounds of this invention can be administered by various methods conventionally used for pharmaceuticals, for example, in such forms of preparation as oral preparations, injections, suppositories, ointments, creams, etc. The dosage forms of the oral preparations may be, for example, tablets, capsules, troches, powders, pills, granules, solutions or suspensions. These dosage forms may contain various additives, for example, binders such as gum arabic, gelatin, sorbitol, tragacanth, poly(vinyl pyrrolidone), poly(vinyl alcohol), hydroxypropyl methyl cellulose, crystalline cellulose, sodium carboxymethyl cellulose, etc.; vehicles such as lactose, sucrose, mannitol, corn starch, calcium phosphate, sorbitol, crystalline

cellulose, etc.; lubricants such as magnesium stearate, talc, polyethylene glycol, silica, etc.; and disintegrators such as potato starch, hydroxypropyl cellulose of low substitution degree, calcium carboxymethyl cellulose, sodium carboxymethyl starch, etc., each alone or in combinations. Soft capsules may also contain conventionally used vehicles, such as vegetable oils, polyethylene glycol, etc., oily suspending agents, liquid preparations and wetting agents such as surfactants.

The liquid preparations may be, for example, oily or aqueous suspensions, syrups, elixirs, and lyophilized products which can be redissolved with water or other additives immediately before use. These liquid preparations may contain suspending agents such as methyl cellulose, sodium carboxymethyl cellulose, hydroxypropyl cellulose, hydroxypropyl methyl cellulose, poly(vinyl pyrrolidone) poly(vinyl alcohol), tragachanth, gelatin, sodium alginate, etc.; emulsifiers such as lecithin, sorbitan, fatty acid esters, gum arabic, tragacanth, etc.; wetting agents such as polyoxyethylene sorbitan fatty acid esters, polyoxyethylene fatty acid esters, hydrogenated castor oil, sesame oil, soybean oil, propylene glycol, polyethylene glycol, ethyl alcohol, etc.; antiseptics such as methyl p-hydroxybenzoate, propyl p-hydroxybenzoate, sorbic acid, etc.; and sweetness such as simple sirups, sucrose, sorbitol, mannitol, etc.; each alone or in combinations.

The rectal preparations may be prepared by using oily bases such as cacao butter, Witepsol, triglyceride, etc., or water-soluble bases such as glycerol, glycerogelatin, Macrogol®, etc.

When the compound of this invention is administered to humans for the purpose of therapy, the dose should be properly adjusted depending on the seriousness of disease, the age and body weight of the patient, and other factors. When the compound is administered to humans for treating autoimmune diseases or nephritis, it is usually administered at a dose of 1-400 mg/man in the form of capsule or tablet 1 to 4 times per day.

Accordingly, the present invention provides use of the compounds of the invention as medicaments. The invention further provides for their use in the manufacture of medicaments for treating kidney disease and autoimmune diseases. Also provided is their use in the manufacture of medicaments for use as enzyme inhibitors, especially where the enzyme is trypsin, plasmin, kallikrein, thrombin or complement.

As an experimental example of treating autoimmune diseases, description is given of an experiment on adjuvant arthritis, and as that of treating nephritis an experiment on Masugi's nephritis.

Experimental Example 1

Effectiveness in treating adjuvant arthritis

The adjuvant arthritis is known as a representative model of autoimmune diseases.

The method of experiment was as follows. A 0.6% Mycobacterium butyricum liquid paraffin suspension, 0.1 ml/animal, was administered to a rat at a subcutaneous site of left hind-limb sole to induce adjuvant arthritis. The present compound was orally administered consecutively from 1 day before the induction to 13 days after the induction. The efficacy of the present compound on adjuvant arthritis was evaluated by examining the swelling accompanied by red spots and subcutaneous knots at the fore-limb, hind-limb, tail, ear and periphery of eye with the lapse of days (on the 8th, 10th, 12th, 14th, 16th and 18th days after adjuvant administration), expressing the results in terms of scores according to the method of F.D. Wood et al. (Int. Arch. Allergy, 35, 456-467 (1969)), and calculating the percentage inhibition relative to the control group. Thus, the higher value of percentage inhibition indicates the higher effectiveness.

The results obtained are shown in Table 2.

Table 2

| Example No. | Dose (mg/kg) | Percentage *) inhibition (%) |
|---|---|---|
| 31 | 30 | 67.7 |
| 17 | 60 | 75.1 |
| 22 | 60 | 97.3 |

Note

*) (Score of animal administered with present compound) ÷ (Score of untreated animal) x 100

Experimental Example 2

Effectiveness in treating Masugi's nephritis

A kidney medulla antiserum of rat, 0.5 ml, obtained by immunizing a rabbit with a homogenate of kidney medulla of a rat together with Freund's complete adjuvant was administered to a rat through the tail vein to induce nephritis.

The present compound was orally administered at a dose of 30 mg/kg. The control compounds used were known compounds I and II shown below selected as those having a relatively strong enzyme inhibitory activity from the compounds described in Japanese Patent Kokai (Laid-open) Nos. 59-139357 and 61-22075, which were orally administered respectively at a dose of 100 mg/kg and compared with the present compounds in effectiveness.

As the index to effectiveness was determined the protein concentration in urine. That is, when inflammation develops in the kidney, it causes damage of renal tubules, etc., and resultant increase in protein concentration in urine.

The results thus obtained are shown in Table 3. As is apparent from the Table, the administration of the present compounds distinctly reduces the protein concentration in urine as compared with non-administered group. Further, it is recognized that while the known compounds are effective at a dose of 100 mg/kg, the present compounds are effective at one third the dose.

Table 3

| Example No. | Dose (mg/kg) | Protein concentration in urine *1) (mg/ml) |
|---|---|---|
| Control group | 0 | 40.8±15.1 |
| 6 | 30 | 19.7±11.1 |
| 11 | 30 | 33.6±17.8 |
| 13 | 30 | 24.2±16.8 |
| 17 | 10 | 2.0±2.0 |
| 22 | 30 | 3.0±2.0 |
| 23 | 30 | 24.2±18.8 |
| 26 | 30 | 25.0±15.4 |
| 32 | 30 | 22.8±13.9 |
| 34 | 30 | 29.3±18.8 |
| 36 | 30 | 26.2±14.7 |
| 38 | 30 | 17.8±12.4 |
| 43 | 30 | 21.8±10.3 |
| 44 | 30 | 20.0±12.9 |
| Known compd. I *2) | 100 | 24.4±11.9 |
| Known compd. II *3) | 100 | 22.1±11.4 |

Note

*1) Expressed in terms of mean value ± standard deviation for 7 animals.

*2) Known compound I (Japanese Patent Kokai (Laid-open) No. 61-22075)

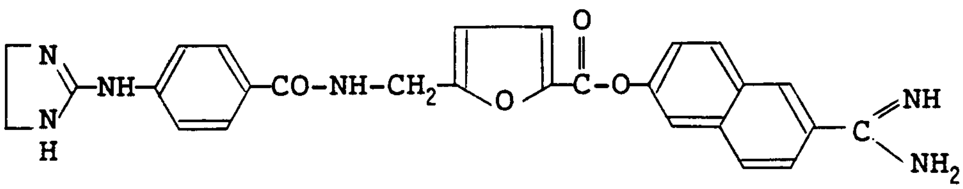

*3) Known compound II (Japanese Patent Kokai (Laid-open) No. 59-139357)

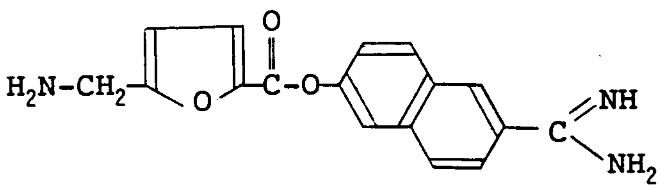

Some examples of pharmaceutical formulations according to this invention are shown below.

Formulation Example 1

| | |
|---|---|
| The present compound | 50 mg |
| Lactose | 23 mg |
| Avicel | 50 mg |
| Carboxycellulose | 25 mg |
| Succinic acid | 50 mg |
| Magnesium stearate | 2 mg |

The total, 200 mg, is filled into a capsule or formed into a tablet.

Formulation Example 2

| | |
|---|---|
| The present compound | 50 mg |
| Lactose | 68 mg |
| Avicel | 50 mg |
| Carboxycellulose | 30 mg |
| Magnesium stearate | 2 mg |

The total, 200 mg, is filled into a capsule or formed into a tablet.

Formulation Example 3

| | |
|---|---|
| The present compound | 50 mg |
| Lactose | 27 mg |
| Corn starch | 27 mg |
| Hydroxypropyl starch | 15 mg |
| Magnesium stearate | 1 mg |

The total, 120 mg, is filled into a capsule or formed into a tablet.

Formulation Example 4

| | |
|---|---|
| The present compound | 50 mg |
| Lactose | 27 mg |
| Corn starch | 27 mg |
| Hydroxypropyl cellulose of low substitution degree | 15 mg |
| Magnesium stearate | 1 mg |

The total, 120 mg, is filled into a capsule or formed into a tablet.

Formulation Example 5

| | |
|---|---|
| The present compound | 50 mg |
| Hydroxypropyl starch | 37 mg |
| Hydroxypropyl cellulose of low substitution degree | 32 mg |
| Magnesium stearate | 1 mg |

The total, 120 mg, is filled into a capsule or formed into a tablet.

This invention is described in more detail below with reference to Examples, but it is in no way limited thereto.

Example 1

6-Amidino-2-naphthyl 5-[4-(2-t-butoxycarbonylvinyl)benzoylaminomethyl]furan-2-carboxylate hydrobromide

In 15 ml of pyridine and 4 ml of N,N-dimethylacetamide (hereinafter abbreviated as DMA) were dissolved 2.0 g of 4-(2-t-butoxycarbonylvinyl)benzoic acid and 0.1 g of 4-dimethylaminopyridine (hereinafter abbreviated as DMAP) and the solution was stirred while cooling with ice. Then, 1.99 g of N,N'-dicyclohexylcarbodiimide (hereinafter abbreviated as DCC) was added to the mixture obtained above. The mixture was stirred at the same temperature for 30 minutes and then 3.49 g of 6-amidino-2-naphthyl 5-aminomethylfuran-2-carboxylate dihydrobromide was added thereto. The reaction mixture was stirred for 3 hours while cooling with ice and then overnight at room temperature, and the precipitated N,N'-dicyclohexylurea (hereinafter abbreviated as DCU) was separated by filtration and washed with a small amount of DMA. The filtrate was added dropwise to 800 ml of ether while stirring and cooling with ice. After the precipitate formed had sedimented, the supernatant was removed by decantation, then 400 ml of acetone was added to the residue and the mixture was stirred for 1 hour at room temperature. After repeating the procedure twice, the precipitated crystals were collected by filtration to obtain 1.88 g of the intended product.

IR$\nu_{max}^{KBr}$ cm$^{-1}$: (-COO-) 1720

NMR (DMSO-$d_6$) δppm:

9.86 - 8.84 (5H, br)

8.72 - 7.30 (12H, m)

6.67 (1H, d, J = 3.5Hz)
6.61 (1H, d, J = 16.4Hz)
4.64 (2H, d, J = 4.4Hz)
1.49 (9H, s)

Example 2

6-Amidino-2-naphthyl 5-[4-(2-carboxyvinyl)-benzoylaminomethyl]furan-2-carboxylate hydrochloride

In 80 ml of acetic acid was dissolved 4 g of 6-amidino-2-naphthyl 5-[4-(2-t-butoxycarbonylvinyl)-benzoylaminomethyl]furan-2-carboxylate hydrochloride. Then, hydrogen chloride gas was bubbled into the resulting solution for 1 hour while stirring and cooling with water. The precipitate was collected by filtration and washed with 120 ml of acetone to obtain 2.8 g of the intended product.

m.p.: 249 - 252.6 °C
$IR\nu_{max}^{KBr}$ $cm^{-1}$: (-COO-) 1705
NMR (DMSO-$d_6$) δppm:
12.12 (1H, br)
10.14 - 8.91 (5H, br)
8.75 - 7.30 (12H, m)
6.67 (1H, d, J = 3.5Hz)
6.65 (1H, d, J = 16.1Hz)
4.63 (2H, d, J = 4.4Hz)

Example 3

6-Amidino-2-naphthyl 5-(4-carboxybenzoyl-aminomethyl)furan-2-carboxylate hydrochloride

To 5 g of terephthalic acid, 3.83 g of 6-amidino-2-naphthyl 5-aminomethylfuran-2-carboxylate dihydrochloride and 0.12 g of DMAP were added 40 ml of N,N-dimethylformamide (hereinafter abbreviated as DMF) and 40 ml of pyridine, the mixture was stirred for 30 minutes while cooling with ice, and then 2.48 g of DCC was added thereto. The mixture was stirred for 1 hour while cooling with ice and for 4 hours while cooling with water. The precipitate was separated by filtration and washed with 5 ml of DMF. The filtrate was added in small portions to 600 ml of ether and the mixture was stirred for 30 minutes at room temperature. Thereafter, the supernatant was removed by decantation and the residue was washed 3 times with 300 ml of acetone with stirring at room temperature.

The crude crystals were dissolved in 36 ml of DMF, then 80 ml of acetone was added to the solution while cooling with water and stirring, and the mixture was stirred for 1 hour. The precipitate was collected by filtration and washed with acetone to obtain 2.2 g of the intended product.

m.p.: 247 - 248.5 °C
$IR\nu_{max}^{KBr}$ $cm^{-1}$: (-COO-) 1720

In the same manner as in Example 1 but by using appropriate starting materials, the respective compounds of the following Examples 4 and 5 were obtained.

Example 4

6-Amidino-2-naphthyl 5-(4-methoxycarbonyl-methylbenzoylaminomethyl)furan-2-carboxylate hydrochloride.

m.p.: 216 - 218 °C
$IR\nu_{max}^{KBr}$ $cm^{-1}$: (-COO-) 1730
NMR (DMSO-$d_6$) δppm:
10.02 - 8.90 (5H, m)
8.81 - 7.09 (11H, m)
6.64 (1H, d, J = 3.5Hz)
4.61 (2H, d, J = 5.6Hz)
3.77 (2H, s)
3.63 (3H, s)

Example 5

6-Amidino-2-naphthyl 5-(4-t-butoxycarbonyl-phenylacetylaminomethyl)furan-2-carboxylate hydrochloride.

m.p.: 178 - 180.5 ° C
IR$\nu_{max}^{KBr}$ $cm^{-1}$: (-COO-) 1730, 1705
NMR (DMSO-$d_6$) δppm:
10.03 - 6.98 (16H, m)
6.56 (1H, d, J = 3.2Hz)
4.42 (2H, d, J = 5.3Hz)
3.61 (2H, s)
1.52 (9H, s)

Example 6

6-Amidino-2-naphthyl 5-(4-carboxyphenylacetyl-aminomethyl)furan-2-carboxylate hydrochloride

To 5.4 g of 6-amidino-2-naphthyl 5-(4-t-butoxycarbonylphenylacetylaminomethyl)furan-2-carboxylate hydrochloride was added 8 ml of acetic acid, and hydrogen chloride gas was passed into the mixture for 2 hours while stirring and cooling with ice. The mixture was further stirred for 2.5 hours with water-cooling, the precipitate was collected by filtration and washed twice with 20 ml of acetone. The crystals were dissolved in 20 ml of DMF, then 1 g of active carbon was added to the solution and stirred for 30 minutes while cooling with ice. The insolubles were removed by filtration, the filtrate was added to 250 ml of acetone and stirred for 2 hours at room temperature. The precipitate was collected by filtration to obtain 3.2 g of the intended product.

m.p.: 232 - 235 ° C
IR$\nu_{max}^{KBr}$ $cm^{-1}$: (-COO-) 1720
NMR (DMSO-$d_6$) δppm:
13.88 - 11.95 (1H, br)
10.37 - 7.17 (16H, m)
6.57 (1H, d, J = 3.5Hz)
4.44 (2H, d, J = 5.0Hz)
3.64 (2H, s)

Example 7

6-Amidino-2-naphthyl 5-(4-carboxymethyl-benzoylaminomethyl)furan-2-carboxylate hydrochloride

To 3 g of 6-amidino-2-naphthyl 5-(4-methoxycarbonylmethylbenzoylaminomethyl)furan-2-carboxylate hydrochloride was added 60 ml of acetic acid, and hydrogen chloride gas was passed into the mixture until saturation while cooling with ice and stirring. Thereafter the mixture was stirred for 48 hours at 50 ° C and then cooled with ice. The precipitate was collected by filtration and washed with 100 ml of acetone. The crystals were dissolved in 40 ml of DMF, then 1 g of active carbon was added to the solution and stirred for 1 hour while cooling with ice. Thereafter, the insolubles were removed by filtration, 90 ml of acetone was added to the filtrate and stirred for 18 hours at room temperature. The precipitate was collected by filtration and washed with 50 ml of acetone to obtain 1.3 g of the intended product.

m.p.: 225 - 228 ° C
IR$\nu_{max}^{KBr}$ $cm^{-1}$: (-COO-) 1730
NMR (DMSO-$d_6$) δppm:
12.41 (1H, br)
10.11 - 8.95 (5H, m)
8.77 - 7.13 (11H, m)
7.60 (1H, d, J = 3.5Hz)
6.65 (1H, d, J = 3.2Hz)
4.62 (2H, d, J = 5.0Hz)
3.94 - 3.51 (2H, m)

Example 8

In the same manner as in Example 6 but by using an appropriate starting material, the following compound was obtained.

6-Amidino-2-naphthyl 5-(4-carboxycinnamoylaminomethyl)furan-2-carboxylate hydrochloride

m.p.: 241 - 245 °C
$IR\nu_{max}^{KBr}$ $cm^{-1}$: (-COO-) 1720
NMR ($DMSO-d_6$) δppm:
13.07 (1H, br)
9.95 - 7.21 (17H, m)
7.15 - 6.43 (2H, m)
4.59 (2H, d, J = 5.9Hz)

Example 9

6-Amidino-2-naphthyl 5-[4-(2-carboxyethyl)-benzoylaminomethyl]furan-2-carboxylate hydrochloride

To a solution of 1.1 g of 6-amidino-2-naphthyl 5-[4-(2-carboxyvinyl)benzoylaminomethyl]furan-2-carboxylate hydrochloride in 18 ml of DMF was added 180 mg of 10% palladium-carbon and the mixture was subjected to catalytic reduction at room temperature for 10 hours. The insolubles were removed by filtration and the filtrate was added dropwise to 300 ml of ether with stirring. The white solid thus precipitated was collected by filtration to obtain 990 mg of the intended product.

m.p.: 208 - 210.5 °C
$IR\nu_{max}^{KBr}$ $cm^{-1}$: (-COO-) 1720
NMR ($DMSO-d_6$) δppm:
12.13 (1H, brs)
10.03 - 8.90 (5H, m)
8.72 - 7.05 (11H, m)
6.63 (1H, d, J = 3.5Hz)
4.60 (2H, d, J = 5.3Hz)
3.15 - 2.28 (4H, m)

Example 10

6-Amidino-2-naphthyl 5-(cis-4-carboxy-1-cyclohexanoylaminomethyl)furan-2-carboxylate hydrochloride

The same procedures as in Example 3 were followed except for using 11.5 g of 6-amidino-2-naphthyl 5-aminomethylfuran-2-carboxylate dihydrochloride and 15.5 g of cis-1,4-cyclohexanedicarboxylic acid, to obtain 5.0 g of the above-mentioned compound in crude form. The crude compound was dissolved in 100 ml of aqueous 90% methanol, 2% aqueous sodium hydrogen-carbonate solution was added thereto, and the precipitated carbonate was collected by filtration and washed with water. The washed product was made into solution by addition of 10 ml of DMF and 0.5 ml of hydrochloric acid, and the solution was added dropwise to 150 ml of acetone while stirring and cooling with ice. The white solid thus precipitated was collected by filtration to obtain 1.3 g of the intended product.

$IR\nu_{max}^{KBr}$ $cm^{-1}$: (-COO-) 1722
NMR ($DMSO-d_6$) δppm:
12.01 (1H, brs)
10.07 - 9.00 (4H, br)
8.80 - 7.25 (8H, m)
6.53 (1H, d, J = 3.5Hz)
4.38 (2H, d, J = 5.0Hz)
2.66 - 0.77 (10H, m)

Example 11

6-Amidino-2-naphthyl 5-(trans-4-carboxy-1-cyclohexanoylaminomethyl)furan-2-carboxylate hydrochloride

In the same manner as in Example 10 but by using an appropriate starting material, the above-mentioned compound was obtained.

m.p.: 242 - 244 °C
IR$\nu_{max}^{KBr}$ $cm^{-1}$: (-COO-) 1740
NMR ($DMSO-d_6$) δppm:
11.76 (2H, br)
10.10 - 9.03 (4H, br)
8.86 - 7.30 (8H, m)
6.54 (1H, d, J = 3.5Hz)
4.39 (2H, d, J = 4.7Hz)
2.69 - 0.79 (10H, m)

Example 12

6-Amidino-2-naphthyl 5-(cis-2-carboxyvinyl)-carbonylaminomethylfuran-2-carboxylate methanesulfonate

In 240 ml of DMF was dissolved 7.7 g of 6-amidino-2-naphthyl 5-aminomethylfuran-2-carboxylate dihydrochloride, and 240 mg of DMAP and 160 ml of pyridine were added thereto. Then, 7 g of maleic acid and 8.2 g of DCC were added to the mixture while stirring and cooling with ice and allowed to react for 2 hours. The precipitate was removed by filtration, the filtrate was added dropwise to 3 ℓ of ether with stirring, the supernatant was removed, then 500 ml of acetone was added to the residue and the resulting precipitate was collected by filtration. The collected precipitate was made into a solution by addition of 40 ml of DMF and 2.0 g of methanesulfonic acid, and the solution was added dropwise to 800 ml of ether with stirring and water-cooling. The supernatant was removed, 50 ml of acetone was added to the residue, then stirred, 800 ml of ether was further added, and the resulting mixture was stirred. The white precipitate was collected by filtration to obtain 2.3 g of the intended product.

IR$\nu_{max}^{KBr}$ $cm^{-1}$: (-COO-) 1710
NMR ($DMSO-d_6$) δppm:
10.05 - 8.86 (5H, m)
8.76 - 7.42 (7H, m)
6.71 (1H, d, J = 3.5Hz)
6.49 (1H, d, J = 12.3Hz)
6.24 (1H, d, J = 12.2Hz)
4.54 (2H, d, J = 5.3Hz)
2.47 (3H, s)

Example 13

6-Amidino-2-naphthyl 5-(trans-2-carboxyvinyl)-carbonylaminomethylfuran-2-carboxylate hydrochloride

In the same manner as in Example 12 but by using an appropriate starting material, the above-mentioned compound was obtained.

IR$\nu_{max}^{KBr}$ $cm^{-1}$: (-COO-) 1730, 1715, 1702
NMR ($DMSO-d_6$) δppm:
10.16 - 8.97 (5H, m)
8.81 - 6.29 (10H, m)
7.03 (1H, d, J = 15.5Hz)
6.67 (1H, d, J = 4.4Hz)
6.57 (1H, d, J = 15.5Hz)
4.54 (2H, d, J = 5.6Hz)

Example 14

6-Amidino-2-naphthyl 5-t-butoxyoxalylaminomethylfuran-2-carboxylate hydrochloride

In a mixture of 160 ml of pyridine and 240 ml of DMF were dissolved 6.5 g of mono-t-butyl oxalate, 7.7 g of 6-amidino-2-naphthyl 5-aminomethylfuran-2-carboxylate dihydrochloride and 250 mg of DMAP, then 8.3 g of DCC was added thereto with stirring and ice-cooling and the mixture was stirred for 24 hours. The precipitate was removed by filtration, the filtrate was added dropwise to 3 ℓ of ether with stirring, the precipitated oily substance was dissolved in a small amount of methanol, and the resulting solution was added dropwise to 2 ℓ of ether with stirring. The precipitate was collected by filtration to obtain 9.0 g of the intended product as a white solid.

IR$\nu_{max}^{KBr}$ $cm^{-1}$: (-COO-) 1730
NMR ($DMSO-d_6$) δppm:
10.14 - 9.08 (5H, m)
9.07 - 6.85 (7H, m)
6.65 (1H, d, J = 3.2Hz)
4.47 (2H, d, J = 5.9Hz)
1.50 (9H, s)

Example 15

6-Amidino-2-naphthyl 5-t-butoxymalonylaminomethylfuran-2-carboxylate hydrochloride

In the same manner as in Example 14 but by using an appropriate starting material, the above-mentioned compound was obtained.

IR$\nu_{max}^{KBr}$ $cm^{-1}$: (-COO-) 1730, 1720
NMR ($DMSO-d_6$) δppm:
10.68 - 9.13 (4H, br)
9.07 - 7.24 (8H, m)
6.63 (1H, d, J = 3.2Hz)
4.43 (2H, d, J = 5.3Hz)
3.23 (2H, s)
1.41 (9H, s)

Example 16

6-Amidino-2-naphthyl 5-oxaloaminomethylfuran-2-carboxylate hydrochloride

In 50 ml of acetic acid was dissolved 7.0 g of 6-amidino-2-naphthyl 5-t-butoxyoxalylaminomethylfuran-2-carboxylate hydrochloride, and hydrogen chloride gas was bubbled into the solution with stirring and water-cooling for 20 minutes. The precipitate was collected by filtration and washed with acetone to obtain 2.7 g of the intended product as a white solid.

m.p.: 210.6 - 215.9 °C
IR$\nu_{max}^{KBr}$ $cm^{-1}$: (-COO-) 1720
NMR ($DMSO-d_6$) δppm:
10.30 - 9.03 (5H, m)
9.00 - 6.97 (8H, m)
6.64 (1H, d, J = 3.5Hz)
4.49 (2H, d, J = 5.5Hz)

Example 17

6-Amidino-2-naphthyl 5-carboxyacetylamino-methylfuran-2-carboxylate hydrochloride

To a mixture of 210 ml of DMF and 90 ml of pyridine were added 10 g of 6-amidino-2-naphthyl 5-aminomethylfuran-2-carboxylate dihydrochloride, 13.6 g of malonic acid and 320 mg of DMAP, then 10.8 g of DCC was added to the mixture with stirring and ice-cooling, and the mixture was stirred for 24 hours. The precipitate was removed by filtration and the filtrate was added dropwise to 3 ℓ of acetone. The precipitate

was collected by filtration to obtain 6.5 g of the intended product as a white solid.

$IR\nu_{max}^{KBr}$ $cm^{-1}$: (-COO-) 1718
NMR ($DMSO-d_6$) δppm:
10.58 - 9.17 (4H, br)
9.17 - 7.17 (8H, m)
6.65 (1H, d, J = 3.5Hz)
4.46 (2H, d, J = 5.9Hz)
3.63 - 2.98 (2H, m)

Example 18

6-amidino-2-naphthyl 5-(3-t-butoxycarbonylpropionylaminomethyl)furan-2-carboxylate hydrochloride.

To 11.4 g of mono-t-butyl succinate, 23.5 g of 6-amidino-2-naphthyl 5-aminomethylfuran-2-carboxylate dihydrochloride and 0.66 g of DMAP was added 115 ml of pyridine, and the mixture was stirred with ice-cooling. After 10 minutes, 25.6 g of DCC was added and the mixture was stirred for 3 hours while cooling with ice and for 16 hours at room temperature. The precipitated crystals were filtered off, and the filtrate was added in small portions into 2 ℓ of ether and stirred at room temperature for 3 hours. After removing the supernatant by decantation, 1 ℓ of acetone was added to the residue and stirred for 1 hour. The precipitate was collected by filtration, then dissolved in 60 ml of methanol and 3 g of active carbon was added to the solution. The mixture was stirred for 30 minutes while cooling with ice, then the insolubles were filtered off and the filtrate was added into 1.5 ℓ of acetone. The mixture was stirred for 5 hours while cooling with ice, and the precipitated crystals were collected by filtration to obtain 21 g of the intended product.

$IR\nu_{max}^{KBr}$ $cm^{-1}$: (-COO-) 1720
NMR ($DMSO-d_6$) δppm:
9.43 (4H, br)
8.88 - 7.07 (8H, m)
6.59 (1H, d, J = 3.5Hz)
4.40 (2H, d, J = 5.3Hz)
2.42 (4H, s)
1.38 (9H, s)

Example 19

In the same manner as in Example 18 but by using an appropriate starting material, the following compound was obtained.

6-Amidino-2-naphthyl 5-(4-t-butoxycarbonyl-butyrylaminomethyl)furan-2-carboxylate hydrochloride

m.p.: 158.2 - 161.8 °C
$IR\nu_{max}^{KBr}$ $cm^{-1}$: (-COO-) 1730, 1715
NMR ($DMSO-d_6$) δppm:
10.07 - 9.12 (4H, br)
8.98 - 7.27 (8H, m)
6.58 (1H, d, J = 3.5Hz)
4.40 (2H, d, J = 5.9Hz)
2.84 - 0.76 (15H, m)

Example 20

6-amidino-2-naphthyl 5-(4-t-butoxycarbonyl-2-guanidinobutyrylaminomethyl)furan-2-carboxylate hydrochloride hydrobromide

To 4.6 g of 4-t-butoxycarbonyl-2-guanidino-butyric acid hydrochloride, 5.3 g of 6-amidino-2-naphthyl 5-aminomethylfuran-2-carboxylate dihydrobromide and 220 mg of DMAP were added 110 ml of DMA and 36 ml of pyridine and the resulting mixture was stirred while cooling with ice. After 10 minutes, 4 g of DCC was added and the mixture was stirred for 3 hours while cooling with ice and then for 16 hours at room

temperature. The precipitated were filtered off, the filtrate was added in small portions into 3 ℓ of ether and stirred at room temperature for 3 hours. After removing the supernatant by decantation, the residue was dissolved in 60 ml of DMF, then 3 g of active carbon was added thereto, and the mixture was stirred for 30 minutes while cooling with ice. The insolubles were filtered off, and the filtrate was added dropwise into 3 ℓ of ether and stirred for 5 hours while cooling with ice. The crystals thus precipitated were collected by filtration to obtain 6.7 g of the intended product.

IR$\nu_{max}^{KBr}$ cm$^{-1}$: (-COO-) 1720
NMR (DMSO-$d_6$) δppm:
9.80 - 6.86 (17H, m)
6.63 (1H, d, J = 3.8Hz)
4.74 - 4.04 (3H, m)
2.57 - 1.84 (4H, m)
1.38 (9H, s)

Example 21

6-amidino-2-naphthyl 5-(3-carboxypropionylaminomethyl)furan-2-carboxylate hydrochloride

To 22.3 g of succinic acid, 24.0 g of 6-amidino-2-naphthyl 5-aminomethylfuran-2-carboxylate dihydrochloride and 790 mg of DMAP was added 240 ml of pyridine, and the mixture was stirred while cooling with ice. After 10 minutes, 25.9 g of DCC was added and the mixture was stirred for 3 hours while cooling with ice and then for 16 hours at room temperature. The precipitate was filtered off, and the filtrate was added in small portions into 2 ℓ of ether and stirred at room temperature for 3 hours. After removing the supernatant by decantation, 1 ℓ of acetone was added to the residue and stirred for 3 hours. The precipitate was collected by filtration to obtain 24.0 g of the intended product.

m.p.: 218 - 219.8 °C
IR$\nu_{max}^{KBr}$ cm$^{-1}$: (-COO-) 1722
NMR (DMSO-$d_6$) δppm:
12.14 (1H, br)
10.17 - 9.10 (4H, br)
8.92 - 7.37 (8H, m)
6.59 (1H, d, J = 3.5Hz)
4.40 (2H, d, J = 5.9Hz)
2.46 (4H, s)

Example 22

6-Amidino-2-naphthyl 5-(4-carboxybutyrylaminomethyl)furan-2-carboxylate hydrochloride

Preparation Method 1

To 11.3 g of glutaric acid and 6.5 g of 6-amidino-2-naphthyl 5-aminomethylfuran-2-carboxylate dihydrochloride was added 50 ml of pyridine and the resulting mixture was stirred while cooling with ice. After 10 minutes, 4.3 g of DCC was added and the mixture was stirred for 3 hours while cooling with ice and then for 16 hours at room temperature. The precipitate was collected by filtration and washed with acetone. Then, 50 ml of DMF and 10 ml of saturated solution of hydrogen chloride in acetic acid were added to the crystals while cooling with water and stirred for 1 hour. The insolubles were removed by filtration, 3.5 g of active carbon was added to the filtrate and the resulting mixture was stirred for 30 minutes. The insolubles were filtered off and the filtrate was added dropwise into 300 ml of ether and stirred for 5 hours while cooling with ice. The supernatant was decanted, and 300 ml of acetone was added to the residue and stirred for 16 hours. The precipitate formed was collected by filtration and washed with 300 ml of acetone to obtain 3.0 g of the intended product.

m.p.: 189 - 193 °C
IR$\nu_{max}^{KBr}$ cm$^{-1}$: (-COO-) 1708
NMR (DMSO-$d_6$) δppm:
10.21 - 8.98 (4H, br)
8.86 - 7.32 (8H, m)
6.58 (1H, d, J = 3.5Hz)

4.40 (2H, d, J = 6.5Hz)
2.74 - 1.32 (6H, m)

Preparation Method 2

The intended compound can be synthesized in the same manner as in Example 6 by using the compound of Example 19.

Preparation Method 3

To 8 g of 6-amidino-2-naphthyl 5-aminomethylfuran-2-carboxylate dihydrochloride, 3.6 g of glutaric anhydride and 1.72 g of anhydrous sodium acetate was added 50 ml of DMF, and the resulting mixture was stirred at 20 °C for 20 hours.

The insolubles thus precipitated were filtered off, 0.8 g of active carbon was added to the filtrate and the mixture was stirred for 2 hours while cooling with ice. The active carbon was filtered off, the filtrate was slowly added into 900 ml of acetone with stirring and ice-cooling, and stirred at the same temperature for 5 hours.

The precipitate was collected by filtration, then added to 450 ml of water, and the mixture was stirred for 4 hours at 70 °C and then overnight with ice-cooling.

The crystals were collected by filtration, then added to a mixture of 80 ml of DMF and 16 ml of acetic acid containing 8% of hydrogen chloride gas, 0.8 g of active carbon was added thereto, and the mixture was stirred for 2 hours while cooling with ice. The insolubles were filtered off, the filtrate was added into 800 ml of acetone while stirring and cooling with ice, and the mixture was stirred at the same temperature for 5 hours.

The crystals thus precipitated were collected by filtration and washed with 80 ml of acetone to obtain 7.6 g of the intended product.

In the same manner as in Example 21 but by using appropriate starting materials, the compounds of Example 23 to 26 shown below were obtained.

Example 23

6-Amidino-2-naphthyl 5-(5-carboxyvalerylaminomethyl)furan-2-carboxylate hydrochloride

m.p.: 215 - 218 °C
$IR\nu_{max}^{KBr}$ $cm^{-1}$: (-COO-) 1725
NMR ($DMSO-d_6$) δppm:
10.06 - 8.97 (4H, br)
8.90 - 7.21 (8H, m)
6.57 (1H, d, J = 3.5Hz)
4.39 (2H, d, J = 5.9Hz)
2.58 - 1.13 (8H, m)

Example 24

6-Amidino-2-naphthyl 5-(6-carboxyhexanoylaminomethyl)furan-2-carboxylate hydrochloride

m.p.: 199 - 201 °C
$IR\nu_{max}^{KBr}$ $cm^{-1}$: (-COO-) 1715
NMR ($DMSO-d_6$) δppm:
10.12 - 6.92 (12H, m)
6.57 (1H, d, J = 3.5Hz)
4.40 (2H, d, J = 5.3Hz)
2.63 - 0.48 (10H, m)

Example 25

6-amidino-2-naphthyl 5-(7-carboxyheptanoylaminomethyl)furan-2-carboxylate hydrochloride

m.p.: 179 - 182 °C
$IR\nu_{max}^{KBr}$ $cm^{-1}$: (-COO-) 1725, 1710
NMR ($DMSO-d_6$) δppm:
10.11 - 7.20 (12H, m)
6.57 (1H, d, J=3.5Hz)
4.40 (2H, d, J=5.3Hz)
2.65 - 0.71 (12H, m)

Example 26

6-Amidino-2-naphthyl 5-(8-carboxyoctanoylaminomethyl)furan-2-carboxylate hydrochloride

m.p.: 176 - 181 °C
$IR\nu_{max}^{KBr}$ $cm^{-1}$: (-COO-) 1735, 1710
NMR ($DMSO-d_6$) δppm:
11.98 (1H, brs)
10.02 - 9.07 (4H, br)
8.82 - 7.34 (8H, m)
6.56 (1H, d, J=3.5Hz)
4.39 (2H, d, J=5.9Hz)
2.46 - 0.82 (14H, m)

Example 27

6-Amidino-2-naphthyl 5-(3-carboxypropionylaminomethyl)furan-2-carboxylate hydrobromide

To 250 g of a 30% hydrobromic acid solution in acetic acid was added with water-cooling 8.0 g of 6-amidino-2-naphthyl 5-(3-t-butoxycarbonylpropionylaminomethyl)furan-2-carboxylate hydrobromide, and the mixture was stirred for 3 hours. The precipitate was filtered off, and the filtrate was poured into 2 ℓ of ether. The mixture was stirred for 3 hours at room temperature and the precipitate was collected by filtration to obtain 5 g of the intended product.

m.p.: 218 - 220 °C

In the same manner as in Example 27 but by using appropriate starting materials, the compounds of Examples 28 and 29 shown below were obtained.

Example 28

6-amidino-2-naphthyl 5-(3-carboxypropionylaminomethyl)furan-2-carboxylate methanesulfonate

m.p.: 175 - 177 °C
$IR\nu_{max}^{KBr}$ $cm^{-1}$: (-COO-) 1720
NMR ($DMSO-d_6$) δppm:
9.79 - 8.88 (4H, br)
8.74 - 7.37 (8H, m)
6.59 (1H, d, J=3.2Hz)
4.40 (2H, d, J=4.7Hz)
2.44 (4H, s)
2.40 (3H, s)

Example 29

6-Amidino-2-naphthyl 5-(4-carboxy-2-guanidinobutyrylaminomethyl)furan-2-carboxylate dihydrobromide

$IR\nu_{max}^{KBr}$ $cm^{-1}$: (-COO-) 1720
NMR ($DMSO-d_6$) δppm:
11.10 - 9.74 (1H, br)
9.65 - 6.80 (16H, m)
6.63 (1H, d, J=3.5Hz)
4.67 - 4.12 (3H, m)
2.73 - 1.56 (4H, m)

Example 30

6-amidino-2-naphthyl 5-(3-carboxypropionylaminomethyl)furan-2-carboxylate aluminium hydroxide hydrochloride

In 1.84 ℓ of 95% ethanol was dissolved 18.4 g of 6-amidino-2-naphthyl 5-(3-carboxypropionylaminomethyl)furan-2-carboxylate hydrochloride, and a solution of 8.42 g of aluminium isopropoxide in 670 ml of toluene was added dropwise thereto at room temperature. After completion of the addition, the reaction mixture was stirred for 3 hours and allowed to stand overnight. The crystals thus precipitated were collected by filtration to obtain 19.1 g of the intended product.
$IR\nu_{max}^{KBr}$ $cm^{-1}$: 3310
1724, 1632, 1560, 1518
1300, 1208, 1145

Example 31

6-Amidino-2-naphthyl 5-(3-N,N-dimethylcarbamoylmethoxycarbonylpropionylaminomethyl)furan-2-carboxylate hydrochloride

To 3.0 g of 3-N,N-dimethylcarbamoylmethoxycarbonylpropionic acid, 3.8 g of 6-amidino-2-naphthyl 5-aminomethylfuran-2-carboxylate dihydrochloride and 0.1 g of DMAP was added 30 ml of pyridine, and the mixture was stirred while cooling with ice. After 10 minutes, 6.2 g of DCC was added and the mixture was stirred for 3 hours while cooling with ice and then for 16 hours at room temperature. The precipitate was filtered off, the filtrate was added in small portions into 700 ml of ether and then the mixture was stirred for 30 minutes. The supernatant was decanted, 700 ml of acetone was added to the residue, then stirred for 1 hour and the supernatant was again removed by decantation. The residue was dissolved in 20 ml of methanol, 0.5 g of active carbon was added thereto, the mixture was stirred for 30 minutes while cooling with ice, and the insoluble was filtered off. The filtrate was added into 700 ml of acetone and stirred for 3 hours while cooling with ice. The precipitate was collected by filtration to obtain 1.4 g of the intended product.
$IR\nu_{max}^{KBr}$ $cm^{-1}$: (-COO-) 1730
NMR ($DMSO-d_6$) δppm:
10.03 - 9.07 (4H, br)
9.02 - 7.12 (8H, m)
6.65 (1H, d, J=3.5Hz)
4.76 (2H, s)
4.41 (2H, d, J=5.6Hz)
2.91 (3H, s)
2.81 (3H, s)
2.72 - 2.31 (4H, m)

In the same manner as in Example 31 but by using appropriate starting materials, the compounds of Examples 32 and 33 shown below were obtained.

Example 32

6-Amidino-2-naphthyl 5-(3-carbamoylpropionylaminomethyl)furan-2-carboxylate hydrochloride

m.p.: 205 - 209 ° C
IR$\nu_{max}^{KBr}$ $cm^{-1}$: (-COO-) 1730, 1720
NMR (DMSO-$d_6$) δppm:
9.56 (4H, br)
8.88 - 6.14 (11H, m)
6.60 (1H, d, J = 3.5Hz)
4.39 (2H, d, J = 5.6Hz)
2.37 (4H, s)

Example 33

6-Amidino-2-naphthyl 5-{[3-(4-methyl-1,3-dioxole-2-one-5-yl)methoxycarbonyl]propionylaminomethyl}furan-2-carboxylate hydrochloride

m.p.: 126 - 130 ° C
IR$\nu_{max}^{KBr}$ $cm^{-1}$: 1810, 1730
NMR (DMSO-$d_6$) δppm:
9.62 (4H, brs)
8.98 - 7.11 (8H, m)
6.58 (1H, d, J = 3.5Hz)
4.94 (2H, s)
4.39 (2H, d, J = 5.6Hz)
2.54 (4H, brs)
2.15 (3H, s)

Example 34

6-Amidino-2-naphthyl 5-(3-methoxycarbonylpropionylaminomethyl)furan-2-carboxylate hydrochloride

To a solution of 18 g of 6-amidino-2-naphthyl 5-aminomethylfuran-2-carboxylate dihydrochloride in 380 ml of DMF was added 19.1 ml of pyridine and then, while cooling with ice, 20 ml of a benzene solution containing 7.5 ml of 3-methoxycarbonylpropionyl chloride was added dropwise thereto for 20 minutes. After stirring at room temperature for 2.5 hours, the reaction mixture was added to 2 ℓ of ether while stirring and cooling with ice, and the mixture was stirred at the same temperature for 5 hours. The supernatant was decanted and 1.5 ℓ of ether was further added and stirred at room temperature for 18 hours. The precipitate was collected by filtration, 800 ml of acetone was added thereto, the mixture was stirred at room temperature for 5 hours, and the crystals were collected by filtration to obtain 19.6 g of the intended product.

m.p.: 198 - 203 ° C
NMR (DMSO-$d_6$) δppm:
10.13 - 9.24 (4H, br)
9.02 - 7.39 (8H, m)
6.59 (1H, d, J = 3.5Hz)
4.41 (2H, d, J = 5.6Hz)
3.59 (3H, s)
2.52 (4H, s)

Example 35

6-Amidino-2-naphthyl 5-(3-t-butoxycarbonylmethylcarbamoylpropionylaminomethyl)furan-2-carboxylate hydrochloride

To 0.75 g of glycine t-butyl ester hydrochloride, 0.15 g of DMAP and 1.5 g of 6-amidino-2-naphthyl 5-(3-carboxypropionylaminomethyl)furan-2-carboxylate hydrochloride were added 20 ml of DMF and 1 ml of

pyridine, and the mixture was stirred while cooling with ice. After 10 minutes, 0.9 g of DCC was added thereto, and the mixture was stirred for 3 hours while cooling with ice and then for 24 hours at room temperature. The precipitate was filtered off, the filtrate was added in small portions to 300 ml of ether, and the mixture was stirred at room temperature for 3 hours. The supernatant was decanted, then 200 ml of ether was added to the residue and stirred at room temperature for 19 hours. The precipitate was collected by filtration, then dissolved in 15 ml of methanol, 0.4 g of active carbon was added thereto and the mixture was stirred for 15 minutes while cooling with ice. The insoluble was filtered off, the filtrate was added to 200 ml of ether and the mixture was stirred for 5 hours while cooling with ice. The precipitate was collected by filtration to obtain 1.4 g of the intended product.

m.p.: 174 - 176 °C
NMR (DMSO-$d_6$) δppm:
10.11 - 9.19 (4H, br)
8.88 - 7.40 (9H, m)
6.59 (1H, d, J = 3.5Hz)
4.40 (2H, d, J = 5.3Hz)
3.70 (2H, d, J = 3.7Hz)
2.42 (4H, s)
1.40 (9H, s)

Example 36

6-Amidino-2-naphthyl 5-(3-carboxymethylcarbamoylpropionylaminomethyl)furan-2-carboxylate hydrochloride

In 35 ml of acetic acid and 3 ml of DMF was dissolved 1.3 g of 6-amidino-2-naphthyl 5-(3-t-butoxycarbonylmethylcarbamoylpropionylaminomethyl)furan-2-carboxylate hydrochloride, and hydrogen chloride gas was passed into the solution for 1 hour while cooling with ice.

The reaction mixture was stirred for 2 hours with ice-cooling and then for 18 hours with water-cooling, then the insoluble was filtered off, the filtrate was added to 300 ml of ether and the mixture was stirred for 3 hours at room temperature. The supernatant was decanted, 200 ml of acetone was added to the residue and the mixture was stirred for 18 hours at room temperature. The precipitate was collected by filtration to obtain 1.1 g of the intended product.

m.p.: 217 - 218 °C
NMR (DMSO-$d_6$) δppm:
10.03 - 9.17 (4H, br)
8.92 - 7.32 (9H, m)
6.61 (1H, d, J = 3.2Hz)
4.60 - 4.10 (2H, m)
3.76 (2H, d, J = 5.4Hz)
2.44 (4H, s)

The same intended product can be also obtained in the following manner. In 35 ml of DMF was dissolved 4 g of the compound of Example 46, then 0.7 g of 5% palladium-carbon was added thereto, and the mixture was subjected to catalytic reduction at room temperature. After 15 hours, the insoluble was filtered off, the filtrate was added slowly to 400 ml of ether, and the mixture was stirred for 3 hours while cooling with ice. The supernatant was decanted, then 1 ml of concentrated hydrochloric acid and 200 ml of ether were added to the residue and stirred for 15 hours while cooling with water. The supernatant was decanted, 200 ml of acetone was added to the residue and the mixture was stirred for 3 hours at room temperature. The precipitate was collected by filtration to obtain 3.1 g of the intended product.

In the same manner as in Example 21 but by using appropriate starting materials, the respective compounds of Examples 37 to 39 shown below were obtained.

Example 37

6-Amidino-2-naphthyl 5-(2-carboxypropionylaminomethyl)furan-2-carboxylate hydrochloride

m.p.: 236 - 239 °C
IR$\nu_{max}^{KBr}$ $cm^{-1}$: (-COO-) 1723
NMR (DMSO-$d_6$) δppm:
10.14 - 9.09 (4H, br)

9.06 - 7.32 (8H, m)
6.58 (1H, d, J = 3.5Hz)
4.39 (2H, d, J = 5.3Hz)
2.51 - 1.85 (1H, m)
1.43 - 0.77 (3H, m)

Example 38

6-Amidino-2-naphthyl 5-(2-carboxybutyrylaminomethyl)furan-2-carboxylate hydrochloride

m.p.: 225 - 228 °C
IR$\nu_{max}^{KBr}$ $cm^{-1}$: (-COO-) 1725
NMR (DMSO-$d_6$) δppm:
10.15 - 9.10 (4H, br)
9.04 - 7.24 (8H, m)
6.57 (1H, d, J = 3.5Hz)
4.40 (2H, d, J = 5.9Hz)
2.36 - 0.65 (6H, m)

Example 39

6-Amidino-2-naphthyl 5-[2-carboxy-2-methylpropionylaminomethyl)furan-2-carboxylate hydrochloride

m.p.: 216 - 220 °C
IR$\nu_{max}^{KBr}$ $cm^{-1}$: (-COO-) 1733
NMR (DMSO-$d_6$) δppm:
10.06 - 9.09 (4H, br)
8.88 - 7.33 (8H, m)
6.54 (1H, d, J = 3.5Hz)
4.39 (2H, d, J = 5.9Hz)
1.10 (3H, s)
0.98 (3H, s)

Example 40

6-Amidino-2-naphthyl 5-[(1-carboxycyclopropan-1-yl)carbonylaminomethyl]furan-2-carboxylate methanesulfonate

In the same manner as in Example 21 but by using an appropriate starting material, 6-amidino-2-naphthyl 5-[(1-carboxycyclopropan-1-yl)carbonylaminomethyl]furan-2-carboxylate hydrochloride was obtained. Then, 0.5 g of the hydrochloride obtained above was dissolved in 3 ml of DMF, and 0.33 ml of triethylamine was added thereto while cooling with ice and stirring. The reaction mixture was stirred for 2 hours while cooling with ice and for 15 hours while cooling with water. The precipitate was collected by filtration, washed with 5 ml of water and dried. Thereafter, 1 ml of DMF and 0.05 g of methanesulfonic acid were added to dissolve the crystals, and 50 ml of ether was added to the solution while stirring and cooling with ice.

After 2 hours, the supernatant was decanted, 70 ml of ether was further added to the residue and the mixture was stirred for 3 hours while cooling with water. The crystals were collected by filtration and washed with 20 ml of ether to obtain 0.16 g of the intended product.

NMR (DMSO-$d_6$) δppm:
9.95 - 8.84 (5H, m)
8.77 - 7.29 (7H, m)
6.59 (1H, d, J = 3.2Hz)
4.51 (2H, d, J = 4.9Hz)
2.41 (3H, s)
1.41 (4H, s)

Example 41

6-Amidino-2-naphthyl 5-(3-carboxy-2,3-dimethylpropionylaminomethyl)furan-2-carboxylate hydrochloride

To 2.3 g of 2,3-dimethylsuccinic acid, 2.0 g of 6-amidino-2-naphthyl 5-aminomethylfuran-2-carboxylate dihydrochloride and 66 mg of DMAP was added 25 ml of pyridine, and the mixture was stirred while cooling with ice. After 10 minutes, 2.16 g of DCC was added and the mixture was stirred for 1 hour at 4 - 8 °C and then for 2 hours at 16 - 17 °C. The precipitate was filtered off, the filtrate was added in small portions into 150 ml of ether and the mixture was stirred for 1 hour at room temperature. The precipitate was collected by filtration, then 60 ml of acetone was added thereto and the mixture was stirred for 3 hours. The precipitate was collected by filtration to obtain 1.3 g of the intended product.

m.p.: 145 °C (d)
$IR\nu_{max}^{KBr}$ $cm^{-1}$: (-COO-) 1720
NMR ($DMSO-d_6$) δppm:
12.58 - 11.87 (1H, br)
10.15 - 9.00 (4H, br)
8.94 - 7.30 (8H, m)
6.57 (1H, d, J = 3.2Hz)
4.43 (2H, d, J = 4.4Hz)
2.97 - 2.24 (2H, m)
1.09 (6H, d, J = 4.7Hz)

Example 42

6-Amidino-2-naphthyl 5-[(2-carboxy-trans-DL-cyclopentan-1-yl)carbonylaminomethyl]furan-2-carboxylate hydrochloride

To 60 ml of pyridine were added 1.9 g of 6-amidino-2-naphthyl 5-aminomethylfuran-2-carboxylate dihydrochloride, 3.9 g of trans-DL-1,2-cyclopentanedicarboxylic acid and 60 mg of DMAP, then, while stirring and cooling with ice, 1.5 g of DCC was added and the mixture was stirred for 24 hours. The precipitate was filtered off and the filtrate was added dropwise into 700 ml of ether. The supernatant was decanted, the residue was dissolved in 70 ml of DMF, and 3.4 ml of triethylamine was added to the solution while stirring and cooling with ice. After stirring for 1 hour, 40 ml of water was added and the mixture was stirred for 10 minutes. The precipitate was collected by filtration, washed with small amounts of water and acetone, and air-dried. Then, 50 ml of acetone was added to the dried product, 1 ml of concentrated hydrochloric acid was further added with ice-cooling and stirring, then, 10 minutes later, 150 ml of ether was added and the mixture was stirred for 24 hours. The precipitate was collected by filtration to obtain 1.5 g of the intended product as a white solid.

m.p.: 151 °C ~ (d)
$IR\nu_{max}^{KBr}$ $cm^{-1}$: (-COO-) 1710
NMR ($DMSO-d_6$) δppm:
10.06 - 9.01 (4H, br)
8.94 - 7.29 (8H, m)
6.54 (1H, d, J = 3.5Hz)
4.40 (2H, d, J = 4.7Hz)
2.97 (2H, brs)
1.71 (6H, brs)

Example 43

In the same manner as in Example 21 but by using an appropriate starting material, the following compound was obtained.

6-Amidino-2-naphthyl 5-(4-carboxy-3-methylbutyrylaminomethyl)furan-2-carboxylate hydrochloride

m.p.: 180 - 181 °C
$IR\nu_{max}^{KBr}$ $cm^{-1}$: (-COO-) 1720
NMR ($DMSO-d_6$) δppm:

10.32 - 9.05 (4H, br)
9.05 - 7.23 (8H, m)
6.57 (1H, d, J = 3.5Hz)
4.40 (2H, d, J = 5.0Hz)
2.77 - 1.71 (5H, m)
0.91 (3H, d, J = 4.4Hz)

Example 44

In the same manner as in Example 42 but by using an appropriate starting material, the following compound was obtained.

6-amidino-2-naphthyl 5-(4-carboxy-3-cyclopentylidenebutyrylaminomethyl)furan-2-carboxylate hydrochloride

m.p.: 180 - 184 °C
IR$\nu_{max}^{KBr}$ $cm^{-1}$: (-COO-) 1735 (sch) 1724
NMR (DMSO-$d_6$) δppm:
12.06 (1H, brs)
10.11 - 8.96 (4H, br)
8.93 - 7.20 (8H, m)
6.56 (1H, d, J = 3.5Hz)
4.41 (2H, d, J = 4.6Hz)
2.44 (2H, s)
2.38 (2H, s)
1.57 (8H, s)

Example 45

In the same manner as in Example 40 but by using an appropriate starting material, the following compound was obtained.

6-Amidino-2-naphthyl 5-(4-carboxy-3-cyclohexylidenebutyrylaminomethyl)furan-2-carboxylate methanesulfonate.

IR$\nu_{max}^{KBr}$ $cm^{-1}$: (-COO-) 1740, 1710
NMR (DMSO-$d_6$) δppm:
9.77 - 8.85 (4H, br)
8.71 - 7.50 (8H, m)
6.58 (1H, d, J = 3.2Hz)
4.45 (2H, d, J = 3.5Hz)
2.64 - 1.94 (7H, m)
1.43 (10H, br)

Example 46

6-Amidino-2-naphthyl 5-(3-benzyloxycarbonylmethylcarbamoylpropionylaminomethyl)furan-2-carboxylate hydrochloride

The intended compound was obtained in the same manner as in Example 35 but by using an appropriate starting material.
NMR (DMSO-$d_6$) δppm:
9.81 - 9.03 (4H, br)
8.78 - 6.94 (14H, m)
6.59 (1H, d, J = 3.2Hz)
5.12 (2H, s)
4.20 (2H, d, J = 4.9Hz)
3.90 (2H, d, J = 5.6Hz)
2.44 (4H, s)

Example 47

6-Amidino-2-naphthyl 5-(4-carboxybutyrylaminomethyl)furan-2-carboxylate methanesulfonate

To 10 ml of DMF was added 2.0 g of 6-amidino-2-naphthyl 5-(4-carboxybutyrylaminomethyl)furan-2-carboxylate hydrochloride, then 4.6 g of methanesulfonic acid was added dropwise while cooling with ice, and the mixture was stirred for 10 minutes. Then, the mixture was added in small portions into 400 ml of acetone and stirred for 4 hours while cooling with ice. The supernatant was decanted, 1 ℓ of ether was added to the residue and stirred, and the precipitate was collected by filtration to obtain 1.54 g of the intended product.

m.p.: 181 - 184 °C
IR$\nu_{max}^{KBr}$ $cm^{-1}$: (-COO-) 1725
NMR (DMSO-$d_6$) δppm:
10.40 - 8.86 (4H, br)
8.80 - 7.18 (8H, m)
6.57 (1H, d, J = 3.5Hz)
4.44 (2H, d, J = 5.6Hz)
2.59 - 1.38 (9H, m)

Example 48

In the same manner as in Example 47 but by using an appropriate starting material, the following compound was obtained.

6-Amidino-2-naphthyl 5-carboxyacetylaminomethylfuran-2-carboxylate methanesulfonate

m.p.: 171 - 172 °C
IR$\nu_{max}^{IBr}$ $cm^{-1}$: (-COO-) 1740
NMR (DMSO-$d_6$) δppm:
12.55 (1H, brs)
9.78 - 8.97 (4H, br)
8.94 - 7.09 (8H, m)
6.63 (1H, d, J = 3.5Hz)
4.44 (2H, d, J = 5.9Hz)
3.24 (1H, s)
2.09 (3H, s)

Example 49

In the same manner as in Example 31 but by using an appropriate starting material, the following compound was obtained.

6-amidino-2-naphthyl 5-(4-carbamoylbutyrylaminomethyl)furan-2-carboxylate hydrochloride

m.p.: 193 - 196 °C
IR$\nu_{max}^{KBr}$ $cm^{-1}$: (-COO-) 1730
NMR (DMSO-$d_6$) δppm:
10.46 - 8.93 (4H, br)
9.07 - 5.99 (11H, m)
4.40 (2H, d, J = 5.3Hz)
2.58 - 1.34 (6H, m)

Example 50

6-amidino-2-naphthyl 5-(19-carboxynonadecanoylaminomethyl)furan-2-carboxylate hydrochloride

The title compound was obtained in the same manner as in Example 42 but by using an appropriate starting material.

m.p.: 188 - 191 °C
$IR\nu_{max}^{KBr}$ $cm^{-1}$: (-COO-) 1738
NMR ($DMSO-d_6$) δppm:
9.85 - 9.30 (4H, br)
8.63 (1H, brs)
8.56 (1H, t, J = 5.6Hz)
8.32 - 8.11 (2H, m)
8.07 - 7.92 (2H, m)
7.73 - 7.52 (2H, m)
6.57 (1H, d, J = 3.3Hz)
4.40 (2H, d, J = 5.6Hz)
2.27 - 2.11 (4H, m)
1.63 - 1.39 (4H, m)
1.22 (28H, brs)

Table 4 shows the structural formulas of the products obtained in the Examples described above.

Table 4

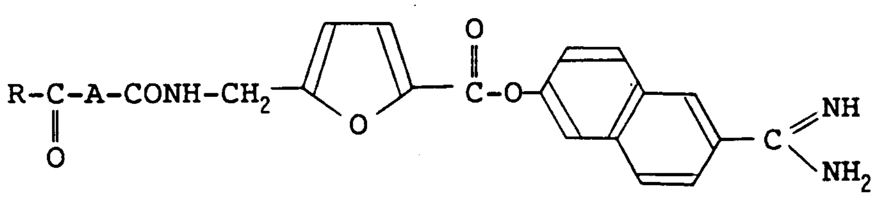

Formula 1

| Example No. | Structural Formula | |
|---|---|---|
| | R | A |
| 1 | 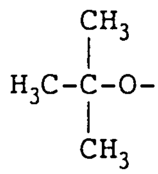 | 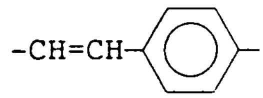 |
| 2 | HO- | 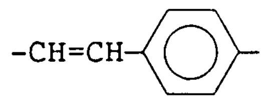 |
| 3 | HO- | 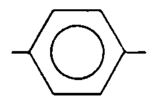 |
| 4 | $H_3CO-$ | 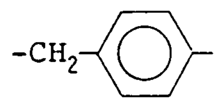 |
| 5 | 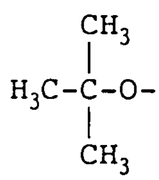 | 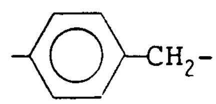 |
| 6 | HO- | 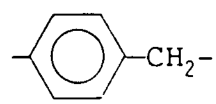 |
| 7 | HO- | 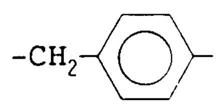 |
| 8 | HO- | 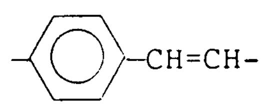 |
| 9 | HO- | 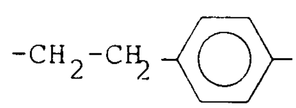 |

| Example No. | R | A |
|---|---|---|
| 10 | HO- | 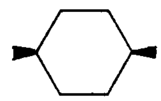 |
| 11 | HO- | 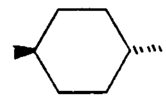 |
| 12 | HO- | 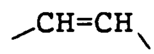 |
| 13 | HO- | 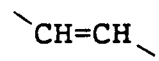 |
| 14 | 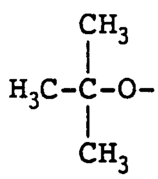 | —— |
| 15 | 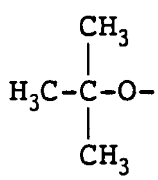 | $-CH_2-$ |
| 16 | HO- | —— |
| 17 | HO- | $-CH_2-$ |
| 18 | 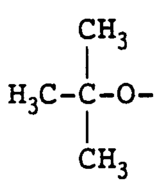 | $-CH_2-CH_2-$ |
| 19 | 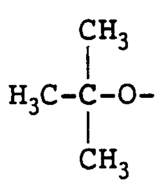 | $-CH_2-CH_2-CH_2-$ |

| Example No. | R | A |
|---|---|---|
| 20 | 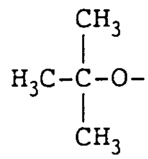 | 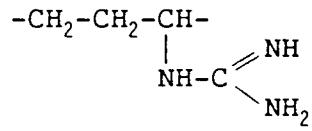 |
| 21 | HO- | $-CH_2-CH_2-$ |
| 22 | HO- | $-(CH_2)_3-$ |
| 23 | HO- | $-(CH_2)_4-$ |
| 24 | HO- | $-(CH_2)_5-$ |
| 25 | HO- | $-(CH_2)_6-$ |
| 26 | HO- | $-(CH_2)_7-$ |
| 27 | HO- | $-CH_2-CH_2-$ |
| 28 | HO- | $-CH_2-CH_2-$ |
| 29 | HO- | 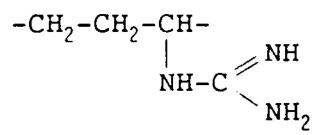 |
| 30 | $(HO)_2AlO-$ | $-CH_2-CH_2-$ |
| 31 | 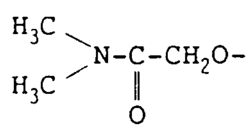 | $-CH_2-CH_2-$ |
| 32 | $H_2N-$ | $-CH_2-CH_2-$ |
| 33 | 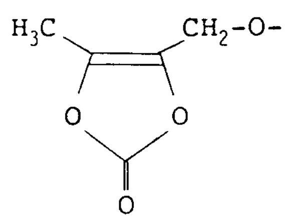 | $-CH_2-CH_2-$ |

| Example No. | R | A |
|---|---|---|
| 34 | $H_3C-O-$ | $-CH_2-CH_2-$ |
| 35 | $CH_3$<br>\|<br>$CH_3-C-O-$<br>\|<br>$CH_3$ | $-CH_2NHCOCH_2CH_2-$ |
| 36 | HO– | $-CH_2NHCOCH_2CH_2-$ |
| 37 | HO– | $CH_3$<br>\|<br>$-CH-$ |
| 38 | HO– | $CH_3-CH_2$<br>\\<br>$-CH-$ |
| 39 | HO– | $CH_3$<br>\|<br>$-C-$<br>\|<br>$CH_3$ |
| 40 | HO– | 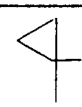 |
| 41 | HO– | $-CH—CH-$<br>\| \|<br>$CH_3$ $CH_3$ |
| 42 | HO– | 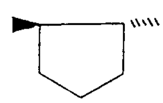 |
| 43 | HO– | $-CH_2-CH-CH_2-$<br>\|<br>$CH_3$ |
| 44 | HO– | 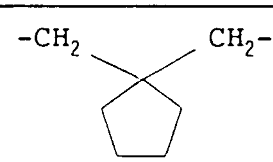 |

| Example No. | R | A |
|---|---|---|
| 45 | HO- | 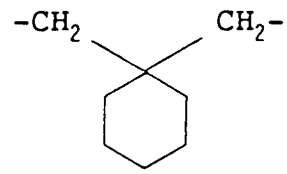 |
| 46 | 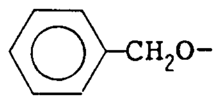 | $-CH_2NHCOCH_2CH_2-$ |
| 47 | HO- | $-(CH_2)_3-$ |
| 48 | HO- | $-CH_2-$ |
| 49 | $H_2N-$ | $-(CH_2)_3-$ |
| 50 | HO- | $-(CH_2)_{18}-$ |

## Claims

**1.** An amidinonapththyl furancarboxylate derivative of the formula I

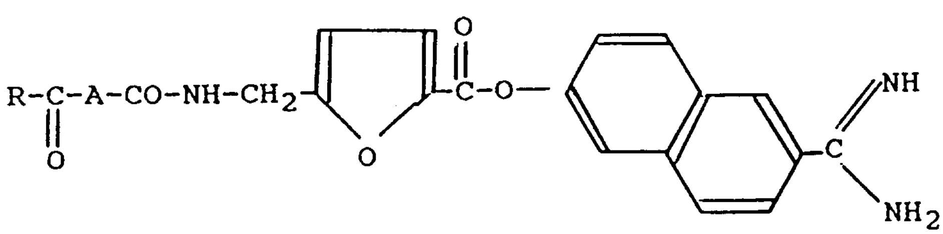

I

wherein A is a single bond or A denotes

a) a phenyl group, a cyclopentyl group or a cyclohexyl group;

b) an alkenylphenyl group, an alkylphenyl group, a phenylalkenyl group or a phenylalkyl group wherein alkyl is $C_1$ to $C_7$ alkyl and alkenyl is $C_2$ to $C_7$ alkenyl;

c) a $C_1$ to $C_{18}$ alkyl or $C_2$ to $C_7$ alkenyl group, which may be substituted by one or two substituents selected from $C_1$ to $C_5$ alkyl groups and guanidino groups, wherein an alkyl substituent together with the carbon atom to which it is attached, may form a cycloalkyl ring having from 3 to 6 carbon atoms, or a $C_1$ to $C_5$ alkyl may itself be substituted by a cycloalkyl ring; or

d) $-(CH_2)_n-NH-CO-(CH_2)_{n'}-$ wherein n and n', which may be the same or different, each represent an integer from 1 to 4; and

R denotes a hydroxyl group; a $C_1$ to $C_7$ alkoxy group which may be mono- or di-substituted by $C_1$ to $C_5$ alkyl; a phenyl-$C_1$-$C_4$alkoxy group; $-OAl(OH)_2$; an amino group;

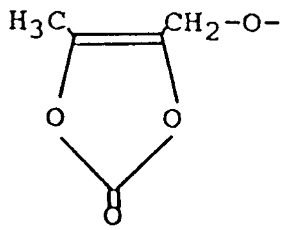

or

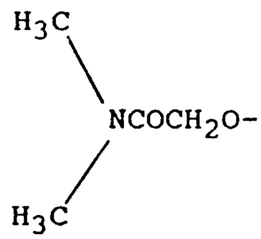

or a pharmaceutically acceptable acid addition salt thereof.

**2.** A derivative according to claim 1 wherein, in the formula I, A is a $C_2$-$C_7$alkenylphenyl group and R is a hydroxyl group or a $C_1$ to $C_7$ alkoxy group which may be mono- or di-substituted by $C_1$ to $C_5$ alkyl, and pharmaceutically acceptable acid addition salts thereof.

**3.** A derivative according to claim 1 wherein, the formula I, A is a phenyl-$C_1$-$C_7$alkyl group and R is a hydroxyl group or a $C_1$ to $C_7$ alkoxy group which may be mono- or di-substituted by $C_1$ to $C_5$ alkyl, and pharmaceutically acceptable acid addition salts thereof.

**4.** A derivative according to claim 1 wherein, in the formula I, A is a $C_1$ to $C_{18}$ alkyl or $C_2$ to $C_7$ alkenyl group, which may be substituted by one or two substituents selected from $C_1$ to $C_5$ alkyl groups and guanidino groups, wherein an alkyl substituent together with the carbon atom to which it is attached, may form a cycloalkyl ring having from 3 to 6 carbon atoms, or a $C_1$ to $C_5$ alkyl may itself be substituted by a cycloalkyl ring, and R is a hydroxyl group; a $C_1$ to $C_7$ alkoxy group which may be mono- or di-substituted by $C_1$ to $C_5$ alkyl; $-OAl(OH)_2$; an amino group,

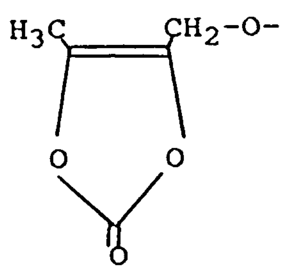

or

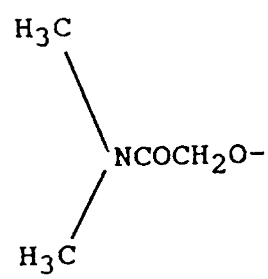

and pharmaceutically acceptable acid addition salts thereof.

**5.** A derivative according to claim 1 wherein, in the formula I, A is an $C_1$-$C_7$alkylphenyl group and R is a hydroxyl group or a $C_1$ to $C_7$ alkoxy group which may be mono- or di-substituted by $C_1$ to $C_5$ alkyl, and pharmaceutically acceptable acid addition salts thereof.

**6.** A derivative according to claim 1 wherein, in the formula I, A is a phenyl group and R is a hydroxyl group, and pharmaceutically acceptable acid addition salts thereof.

**7.** A derivative according to claim 1 wherein, in the formula I, A is a phenyl-$C_2$-$C_7$alkenyl group and R is a hydroxyl group, and pharmaceutically acceptable acid addition salts thereof.

**8.** A derivative according to claim 1 wherein, in the formula I, A is a cyclopentyl or cyclohexyl group and R is a hydroxyl group, and pharmaceutically acceptable acid addition salts thereof.

**9.** A derivative according to claim 1 wherein, in the formula I, A is $-(CH_2)_n$-NH-CO-$(CH_2)_{n'}$- wherein n and n', which may be the same or different, each represent an integer from 1 to 4, and R is a hydroxyl group or a $C_1$ to $C_7$ alkoxy group which may be mono- or di-substituted by $C_1$ to $C_5$ alkyl or a phenyl—$C_1$-$C_4$-alkoxy group, and pharmaceutically acceptable acid addition salts thereof.

**10.** A derivative according to claim 1 wherein, in the formula I, A is a single bond and R is a hydroxyl group or a $C_1$ to $C_7$ alkoxy group which may be mono- or di-substituted by $C_1$ to $C_5$ alkyl, and pharmaceutically acceptable acid addition salts thereof.

**11.** A derivative according to claim 1 wherein, in the formula I, A is a $C_1$ to $C_{18}$ alkyl group and R is a hydroxyl group, amino group or a $C_1$ to $C_7$ alkoxy group, and pharmaceutically acceptable acid addition salts thereof.

**12.** Pharmaceutical preparations comprising the derivatives or the acid addition salts thereof according to any one of claims 1 to 11, optionally in combination with at least one pharmaceutically acceptable carrier.

**13.** A derivative or an acid addition salt thereof according to any one of claims 1 to 11, for use as a medicament.

**14.** Use of a derivative or an acid addition salt thereof according to any one of claims 1 to 11, for the manufacture of a medicament for treating kidney disease.

**15.** Use of a derivative or an acid addition salt thereof according to any one of claims 1 to 11, for the manufacture of a medicament for treating autoimmune diseases.

**16.** Use of a derivative or an acid addition salt thereof according to any one of claims 1 to 11, for the manufacture of a medicament for use as an enzyme inhibitor.

**17.** Use in vitro of a derivative or an acid addition salt thereof according to any one of claims 1 to 11, as an enzyme inhibitor.

**18.** Use of a derivative or an acid addition salt thereof according to claim 16 or claim 17, wherein the enzyme is trypsin, plasmin, kallikrein, thrombin or complement.

**19.** A method for preventing the decomposition of collected blood, which comprises admixing a derivative or acid addition salt thereof according to any one of claims 1 to 11 with the collected blood.

**Patentansprüche**

**1.** Amidinonaphthylfurancarboxylat-Derivat der Formel I

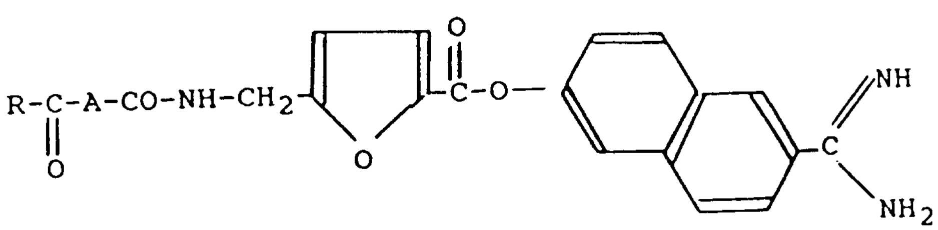

I

worin A eine Einfachbindung ist oder A

a) eine Phenylgruppe, Cyclopentylgruppe oder Cyclohexylgruppe bedeutet;

b) eine Alkenylphenylgruppe, Alkylphenylgruppe, Phenylalkenylgruppe oder Phenylalkylgruppe bedeutet, worin Alkyl für $C_1$-$C_7$-Alkyl und Alkenyl für $C_2$-$C_7$-Alkenyl steht;

c) eine $C_1$-$C_{18}$-Alkyl- oder $C_2$-$C_7$-Alkenylgruppe ist, die durch einen oder zwei Substituenten, ausgewählt unter $C_1$-$C_5$-Alkylgruppen und Guanidinogruppen, substituiert sein kann, worin ein Alkylsubstituent zusammen mit dem Kohlenstoffatom, an welches er gebunden ist, einen Cycloalkylring mit 3 bis 6 Kohlenstoffatomen bilden kann, oder $C_1$-$C_5$-Alkyl durch einen Cycloalkylring substituiert sein kann; oder

d) $-(CH_2)_n-NH-CO-(CH_2)_{n'}-$ bedeutet, worin n und n', die gleich oder verschieden sein können, jeweils eine ganze Zahl von 1 bis 4 bedeuten; und

R eine Hydroxylgruppe; eine $C_1$-$C_7$-Alkoxygruppe, die durch $C_1$-$C_5$-Alkyl mono- oder disubstituiert sein kann; eine Phenyl-$C_1$-$C_4$-alkoxygruppe; -OAl(OH)$_2$; eine Aminogruppe;

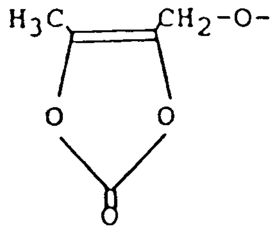

oder

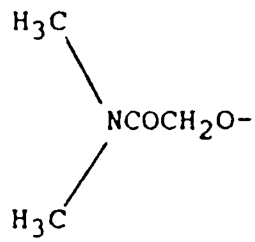

bedeutet; oder ein pharmazeutisch verträgliches Säureadditionssalz davon.

2. Derivat gemäß Anspruch 1, worin in Formel I
A eine $C_2$-$C_7$-Alkenylphenylgruppe und R eine Hydroxylgruppe oder $C_1$-$C_7$-Alkoxygruppe bedeutet, die durch $C_1$-$C_5$-Alkyl mono- oder disubstituiert sein kann, und pharmazeutisch verträgliche Säureadditionssalze davon.

3. Derivat gemäß Anspruch 1, worin in Formel I
A eine Phenyl-$C_1$-$C_7$-alkylgruppe und R eine Hydroxylgruppe oder $C_1$-$C_7$-Alkoxygruppe bedeutet, die durch $C_1$-$C_5$-Alkyl mono- oder disubstituiert sein kann, und pharmazeutisch verträgliche Säureadditionssalze davon.

4. Derivat nach Anspruch 1, worin A in Formel I
A eine $C_1$-$C_{18}$-Alkyl- oder $C_2$-$C_7$-Alkenylgruppe ist, die durch einen oder zwei Substituenten, ausgewählt unter $C_1$-$C_5$-Alkylgruppen und Guanidinogruppen, substituiert sein kann, worin ein Alkylsubstituent zusammen mit dem Kohlenstoffatom, an das er gebunden ist, einen Cycloalkylring mit 3 bis 6 Kohlenstoffatomen bilden kann oder $C_1$-$C_5$-Alkyl selbst mit einem Cycloalkylring substituiert sein kann, und R eine Hydroxylgruppe; eine $C_1$-$C_7$-Alkoxygruppe, die durch $C_1$-$C_5$-Alkyl mono- oder disubstituiert sein kann; -OAl(OH)$_2$; eine Aminogruppe;

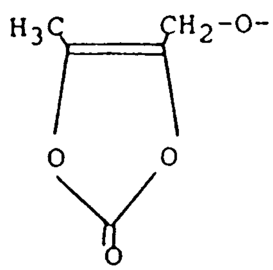

oder

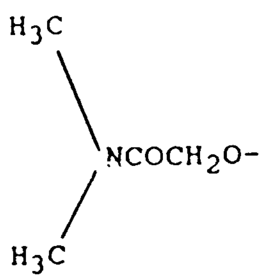

bedeutet; und pharmazeutisch verträgliche Säureadditionssalze davon.

5. Derivat nach Anspruch 1, worin in Formel I
A eine $C_1$-$C_7$-Alkylphenylgruppe und R eine Hydroxylgruppe oder $C_1$-$C_7$-Alkoxygruppe ist, die durch $C_1$-$C_5$-Alkyl mono- oder disubstitiuert sein kann, und pharmazeutisch verträgliche Säureadditionssalze davon.

6. Derivat nach Anspruch 1, worin in Formel I
A eine Phenylgruppe und R eine Hydroxylgruppe ist, und pharmazeutisch verträgliche Säureadditionssalze davon.

7. Derivat nach Anspruch 1, worin in Formel I
A eine Phenyl-$C_2$-$C_7$-alkenylgruppe und R eine Hydroxylgruppe ist, und pharmazeutisch verträgliche Säureadditionssalze davon.

8. Derivat nach Anspruch 1, worin in Formel I
A eine Cyclopentyl- oder Cyclohexylgruppe und R eine Hydroxylgruppe ist, und pharmazeutisch verträgliche Säureadditionssalze davon.

9. Derivat nach Anspruch 1, worin in Formel I
A für $-(CH_2)_n-NH-CO-(CH_2)_{n'}-$ steht, worin n und n', die gleich oder verschieden sein können, jeweils eine ganze Zahl von 1 bis 4 bedeuten, und R eine Hydroxylgruppe oder $C_1$-$C_7$-Alkoxygruppe ist, die durch eine $C_1$-$C_5$-Alkyl- oder Phenyl-$C_1$-$C_4$-alkoxygruppe mono- oder disubstituiert sein kann, und pharmazeutisch verträgliche Säureadditionssalze davon.

10. Derivat nach Anspruch 1, worin in der Formel I
A eine Einfachbindung und R eine Hydroxylgruppe oder eine $C_1$-$C_7$-Alkoxygruppe ist, die durch $C_1$-$C_5$-Alkyl mono- oder disubstituiert sein kann, und pharmazeutisch verträgliche Säureadditionssalze davon.

11. Derivat nach Anspruch 1, worin in der Formel I
A eine $C_1$-$C_{18}$-Alkylgruppe und R eine Hydroxylgruppe, Aminogruppe oder $C_1$-$C_7$-Alkoxygruppe ist, und pharmazeutisch verträgliche Säureadditionssalze davon.

12. Pharmazeutische Mittel, welche die Derivate oder die Säureadditionssalze davon nach einem der Ansprüche 1 bis 11, gegebenenfalls zusammen mit wenigstens einem pharmazeutisch verträglichen Träger, umfassen.

13. Derivat oder Säureadditionssalz davon nach einem der Ansprüche 1 bis 11, zur Verwendung als Medikament.

14. Verwendung eines Derivates oder eines Säureadditionssalzes davon nach einem der Ansprüche 1 bis 11, für die Herstellung eines Medikamentes zur Behandlung einer Nierenerkrankung.

15. Verwendung eines Derivates oder eines Säureadditionssalzes davon nach einem der Ansprüche 1 bis 11, für die Herstellung eines Medikamentes zur Behandlung von Autoimmun-Erkrankungen.

16. Verwendung eines Derivates oder eines Säureadditionssalzes davon nach einem der Ansprüche 1 bis 11, für die Herstellung eines Medikamentes zur Anwendung als Enzyminhibitor.

17. In-vitro-Verwendung eines Derivates oder eines Säureadditionssalzes davon nach einem der Ansprüche 1 bis 11, als Enzyminhibitor.

18. Verwendung eines Derivates oder seines Säureadditionssalzes davon nach Anspruch 16 oder 17, wobei das Enzym Trypsin, Plasmin, Kallikrein, Thrombin oder Komplement ist.

19. Verfahren zur Verhinderung der Zersetzung von gewonnenem Blut, welches das Mischen eines Derivates oder eines Säureadditionssalzes davon nach einem der Ansprüche 1 bis 11 mit dem gewonnenen Blut umfaßt.

## Revendications

1. Dérivé de furanne-carboxylate d'amidinonaphtyle de formule I

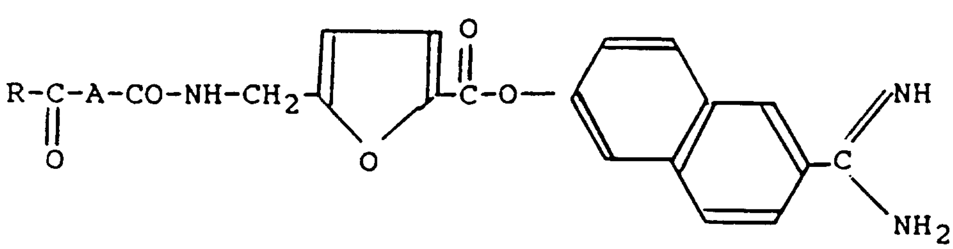

I

où A est une liaison simple ou A représente

a) un groupe phényle, un groupe cyclopentyle ou un groupe cyclohexyle ;

b) un groupe alcénylphényle, un groupe alkylphényle, un groupe phénylalcényle ou un groupe phénylalkyle, où le groupe alkyle est un groupe alkyle en $C_1$ à $C_7$ et le groupe alcényle est un groupe alcényle en $C_2$ à $C_7$ ;

c) un groupe alkyle en $C_1$ à $C_{18}$ ou un groupe alcényle en $C_2$ à $C_7$, qui peuvent être substitués par un ou deux substituants choisis parmi les groupes alkyles en $C_1$ à $C_5$ et les groupes guanidino, où un substituant alkyle avec l'atome de carbone auquel il est attaché peuvent former un cycle cycloalkyle ayant de 3 à 6 atomes de carbone, ou un groupe alkyle en $C_1$ à $C_5$ peut lui-même être substitué par un cycle cycloalkyle ; ou

d) $-(CH_2)_n-NH-CO-(CH_2)_{n'}-$ où n et n', qui peuvent être les mêmes ou différents, représentent chacun un nombre entier de 1 à 4 ; et

R représente un groupe hydroxyle ; un groupe alcoxy en $C_1$ à $C_7$ qui peut être mono- ou disubstitué par un groupe alkyle en $C_1$ à $C_5$ ; un groupe phényl-alcoxy en $C_1$ à $C_4$ ; $-OAl(OH)_2$ ; un groupe amino ;

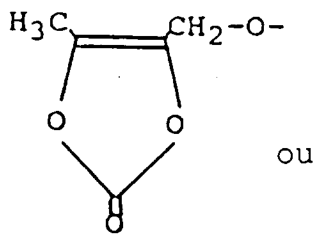

ou

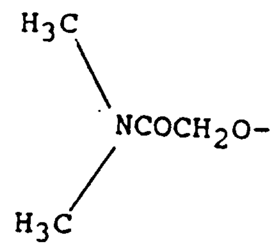

ou un sel pharmaceutiquement acceptable d'addition de celui-ci avec un acide.

**2.** Dérivé selon la revendication 1, dans lequel, dans la formule I, A est un groupe alcényle en $C_2$ à $C_7$-phényle et R est un groupe hydroxyle ou un groupe alcoxy en $C_1$ à $C_7$ qui peut être mono- ou disubstitué par un groupe alkyle en $C_1$ à $C_5$, et les sels pharmaceutiquement acceptables d'addition de celui-ci avec un acide.

**3.** Dérivé selon la revendication 1, dans lequel, dans la formule I, A est un groupe phényl-alkyle en $C_1$ à $C_7$ et R est un groupe hydroxyle ou un groupe alcoxy en $C_1$ à $C_7$ qui peut être mono- ou disubstitué par un groupe alkyle en $C_1$ à $C_5$, et les sels pharmaceutiquement acceptables d'addition de celui-ci avec un acide.

**4.** Dérivé selon la revendication 1, dans lequel, dans la formule I, A est un groupe alkyle en $C_1$ à $C_{18}$ ou un groupe alcényle en $C_2$ à $C_7$, qui peut être substitué par un ou deux substituants choisis parmi les groupes alkyles en $C_1$ à $C_5$ et les groupes guanidino, où un substituant alkyle avec l'atome de carbone auquel il est attaché peuvent former un cycle cycloalkyle ayant de 3 à 6 atomes de carbone, ou un groupe alkyle en $C_1$ à $C_5$ peut lui-même être substitué par un cycle cycloalkyle, et R est un groupe hydroxyle ; un groupe alcoxy en $C_1$ à $C_7$ qui peut être mono- ou disubstitué par un groupe alkyle en $C_1$ à $C_5$ ; $-OAl(OH)_2$ ; un groupe amino,

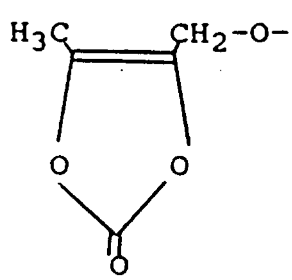

ou

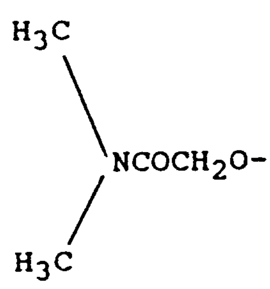

et les sels pharmaceutiquement acceptables d'addition de celui-ci avec un acide.

**5.** Dérivé selon la revendication 1, dans lequel, dans la formule I, A est un groupe alkyle en $C_1$ à $C_7$-phényle et R est un groupe hydroxyle ou un groupe alcoxy en $C_1$ à $C_7$ qui peut être mono- ou disubstitué par un groupe alkyle en $C_1$ à $C_5$, et les sels pharmaceutiquement acceptables d'addition de celui-ci avec un acide.

**6.** Dérivé selon la revendication 1, dans lequel, dans la formule I, A est un groupe phényle et R est un groupe hydroxyle, et les sels pharmaceutiquement acceptables d'addition de celui-ci avec un acide.

**7.** Dérivé selon la revendication 1, dans lequel, dans la formule I, A est un groupe phényl-alcényle en $C_2$ à $C_7$ et R est un groupe hydroxyle, et les sels pharmaceutiquement acceptables d'addition de celui-ci avec un acide.

**8.** Dérivé selon la revendication 1, dans lequel, dans la formule I, A est un groupe cyclopentyle ou cyclohexyle et R est un groupe hydroxyle, et les sels pharmaceutiquement acceptables d'addition de celui-ci avec un acide.

**9.** Dérivé selon la revendication 1, dans lequel, dans la formule I, A est $-(CH_2)_n-NH-CO-(CH_2)_{n'}-$ dans laquelle n et n', qui peuvent être les mêmes ou différents, représentent chacun un nombre entier de 1 à 4, et R est un groupe hydroxyle ou un groupe alcoxy en $C_1$ à $C_7$ qui peut être mono- ou disubstitué par un groupe alkyle en $C_1$ à $C_5$ ou un groupe phényl-alcoxy en $C_1$ à $C_4$, et les sels pharmaceutiquement acceptables d'addition de celui-ci avec un acide.

**10.** Dérivé selon la revendication 1, dans lequel, dans la formule I, A est une liaison simple et R est un groupe hydroxyle ou un groupe alcoxy en $C_1$ à $C_7$ qui peut être mono- ou disubstitué par un groupe alkyle en $C_1$ à $C_5$, et les sels pharmaceutiquement acceptables d'addition de celui-ci avec un acide.

**11.** Dérivé selon la revendication 1, dans lequel, dans la formule I, A est un groupe alkyle en $C_1$ à $C_{18}$ et R est un groupe hydroxyle, un groupe amino ou un groupe alcoxy en $C_1$ à $C_7$, et les sels pharmaceutiquement acceptables d'addition de celui-ci avec un acide.

**12.** Préparations pharmaceutiques comprenant les dérivés ou les sels d'addition de ceux-ci avec un acide selon l'une quelconque des revendications 1 à 11, éventuellement en combinaison avec au moins un véhicule pharmaceutiquement acceptable.

**13.** Dérivé ou sel d'addition de celui-ci avec un acide selon l'une quelconque des revendications 1 à 11, pour l'utilisation en tant que médicament.

**14.** Utilisation d'un dérivé ou d'un sel d'addition de celui-ci avec un acide selon l'une quelconque des revendications 1 à 11, pour la fabrication d'un médicament pour traiter une maladie des reins.

**15.** Utilisation d'un dérivé ou d'un sel d'addition de celui-ci avec un acide selon l'une quelconque des revendications 1 à 11, pour la fabrication d'un médicament pour traiter des maladies auto-immunes.

**16.** Utilisation d'un dérivé ou d'un sel d'addition de celui-ci avec un acide selon l'une quelconque des revendications 1 à 11, pour la fabrication d'un médicament à utiliser en tant qu'inhibiteur d'enzyme.

**17.** Utilisation *in vitro* d'un dérivé ou d'un sel d'addition de celui-ci avec un acide selon l'une quelconque des revendications 1 à 11, en tant qu'inhibiteur d'enzyme.

**18.** Utilisation d'un dérivé ou d'un sel d'addition de celui-ci avec un acide selon la revendication 16 ou la revendication 17, dans laquelle l'enzyme est la trypsine, la plasmine, la kallikréine, la thrombine ou un complément.

**19.** Procédé pour éviter la décomposition de sang prélevé, qui comprend le mélange d'un dérivé ou d'un sel d'addition de celui-ci avec un acide selon l'une quelconque des revendications 1 à 11 avec le sang prélevé.